# EUROPEAN PATENT APPLICATION

(11) **EP 4 755 915 A1**
(43) Date of publication of application: **10.06.2026**
(21) Application number: 24849617.6
(22) Date of filing: 31.07.2024
(51) Int. Cl.: C07K 16/28, A61P 37/00, A61K 39/00

(54) **ANTIBODY AND PHARMACEUTICAL COMPOSITION COMPRISING SAME FOR TREATING IMMUNE DISEASES**

(30) Priority: 31.07.2023 KR 20230100041
(71) Applicant: Medytox Inc., Chungcheongbuk-do 28126 (KR)
(72) Inventor: LEE, Jun Ho, Suwon-si Gyeonggi-do 16280 (KR); JANG, So Young, Suwon-si Gyeonggi-do 16282 (KR); KIM, Ji Hye, Hwaseong-si Gyeonggi-do 18429 (KR); KIM, Hyun Wook, Seongnam-si Gyeonggi-do 13491 (KR)
(74) Representative: Tergau & Walkenhorst Intellectual Property GmbH
(86) International application number: PCT/KR2024/011266
(87) International publication number: WO 2025/029050

(57) **Abstract**

The present invention provides an antibody capable of effectively preventing or treating immune diseases associated with a decrease in the function of an autoantigen by competitively inhibiting autoantibodies without side effects, and a pharmaceutical composition for treating immune diseases comprising the same.

## Description

### Technical Field

The present invention relates to an antibody and a pharmaceutical composition for treating immune diseases comprising the same.

### Background Art

The formation of autoantibodies against substances within the body plays a crucial role in the pathological mechanisms of various autoimmune diseases. In particular, when the antigenic proteins of autoantibodies present on the cell surface form multiple complexes, the binding of the antibody to the antigen leads to cross-linking on the cell surface. Such cross-linking may cause by triggering immune responses against antigen-presenting cells-including complement-dependent cytotoxicity (CDC) through complement activation, antibody-dependent cellular cytotoxicity (ADCC), antibody-dependent cellular phagocytosis (ADCP), and antigen internalization or through the loss of antigen function.

To treat diseases caused by autoantibodies, agents such as IVIg (Intravenous Immunoglobulin), FcRn inhibitors, and complement inhibitors-which act to rapidly remove antibodies from the body-as well as antibodies that eliminate antibody-producing B cells, are currently in use. However, there remains a continuous demand for therapeutic agents capable of specifically inhibiting the pathological effects caused by autoantibodies. In this regard, Korean Patent Publication No. 2020-0098604 discloses an FcRn antibody and methods for its use.

### Detailed Description of the Invention

### Technical Problem

However, the aforementioned prior art poses a problem of inducing numerous side effects, as its mechanism involves suppressing the body's immune response rather than directly eliminating the cause of the disease.

The present invention is intended to resolve various problems, including those mentioned above, and was conducted with the support of the Korea Drug Development Fund (KDDF) funded by the Ministry of Science and ICT, the Ministry of Trade, Industry and Energy, and the Ministry of Health and Welfare (Project Number: RS-2023-00283006). The objective of the present invention is to provide an antibody and a pharmaceutical composition for treating immune diseases comprising the same, which can effectively prevent or treat immune diseases related to the reduction of autoantigen function by competitively inhibiting autoantibodies without side effects. However, these objectives are exemplary, and the scope of the present invention is not limited thereto.

### Technical Solution

According to one aspect of the present invention, there is provided a recombinant homodimeric antibody having specificity for an autoantigen, which is derived from an autoantibody having specificity for the autoantigen and comprises a light chain and a heavy chain,

wherein the heavy chain comprises at least a portion of a light chain variable region (V_{L}) of the autoantibody, and the light chain comprises at least a portion of a heavy chain variable region (V_{H}) of the autoantibody.

According to another aspect of the present invention, there is provided a pharmaceutical composition for preventing or treating autoimmune diseases, comprising the homodimeric antibody and a pharmaceutically acceptable carrier.

According to another aspect of the present invention, there is provided a recombinant dimeric antibody comprising a light chain variable region and a heavy chain variable region and having specificity for an autoantigen,

wherein in at least one monomer of the recombinant dimeric antibody, at least a portion of a light chain variable region (V_{L}) and at least a portion of a heavy chain variable region (V_{H}) of an autoantibody-which has specificity for the autoantigen and has pathogenicity for an autoimmune disease-are exchanged with each other.

### Advantageous Effects of the Invention

The antibody of the present invention and the pharmaceutical composition for treating immune diseases comprising the same, constructed as described above, are capable of inhibiting or eliminating the pathological effects of autoantibodies without inducing side effects. Accordingly, they can be effectively utilized for the prevention or treatment of immune diseases associated with a reduction in autoantigen function. It should be understood, however, that the scope of the present invention is not limited by these effects.

### Brief Description of Drawings

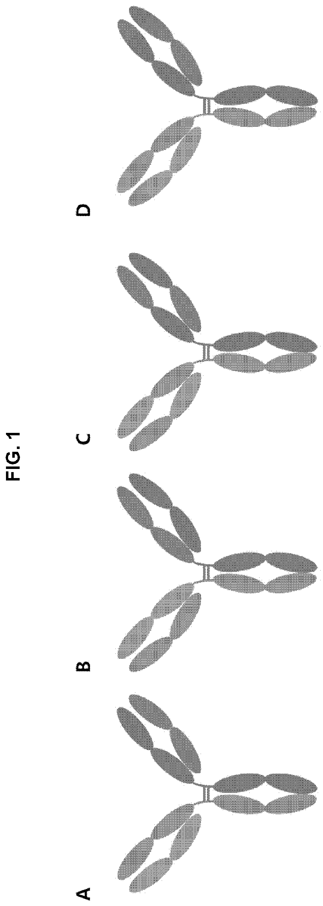

FIG. 1 is a schematic diagram illustrating the structure of an antibody according to an embodiment of the present invention. FIG. 1A illustrates an autoantibody against an autoantigen, and FIGs. 1B to 1D exemplarily illustrate antibodies according to various embodiments.

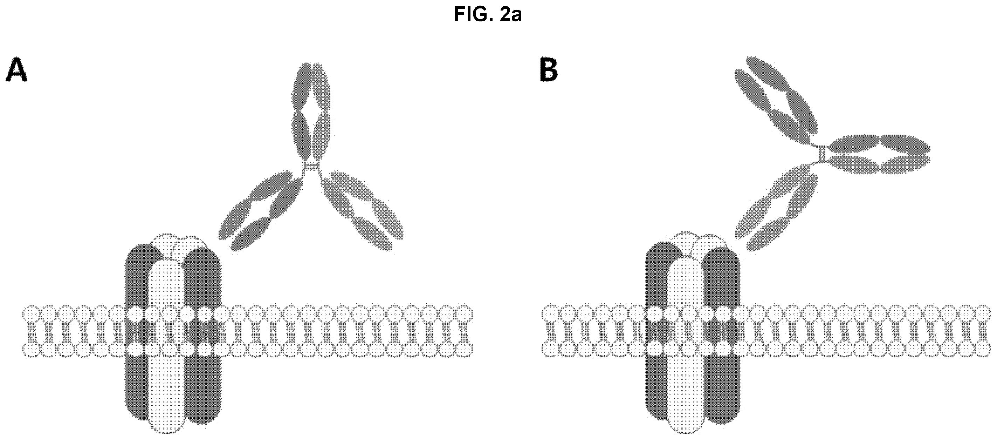

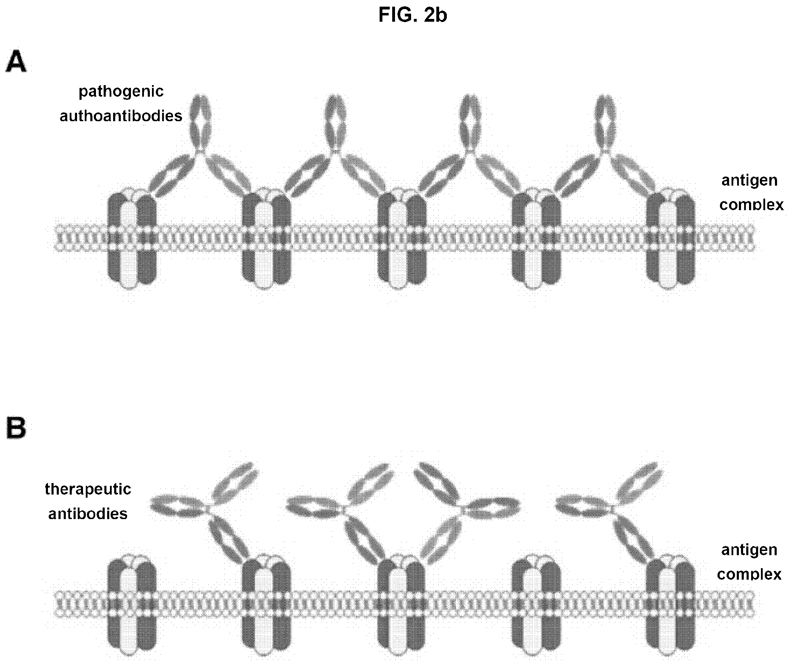

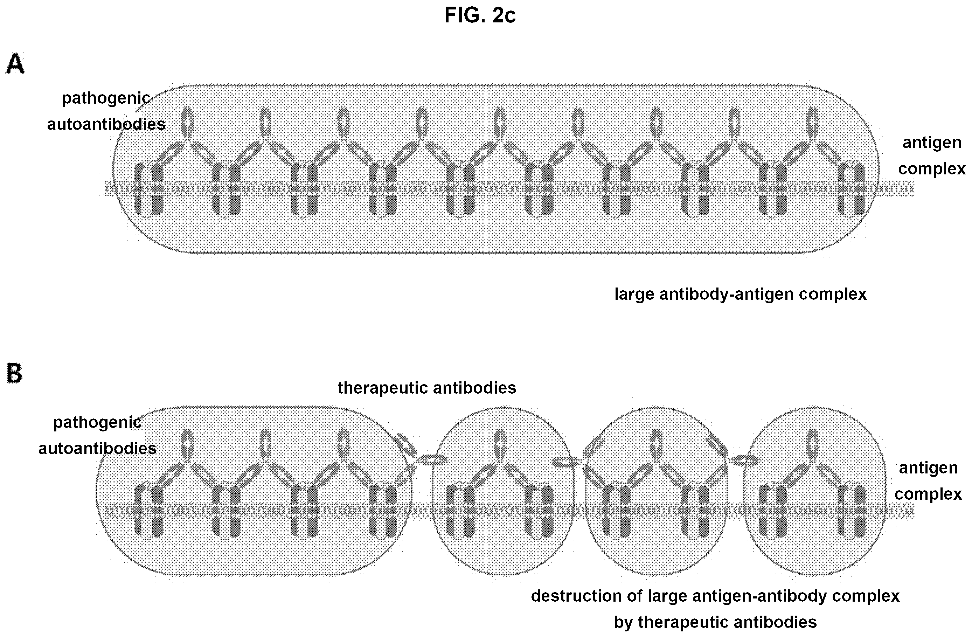

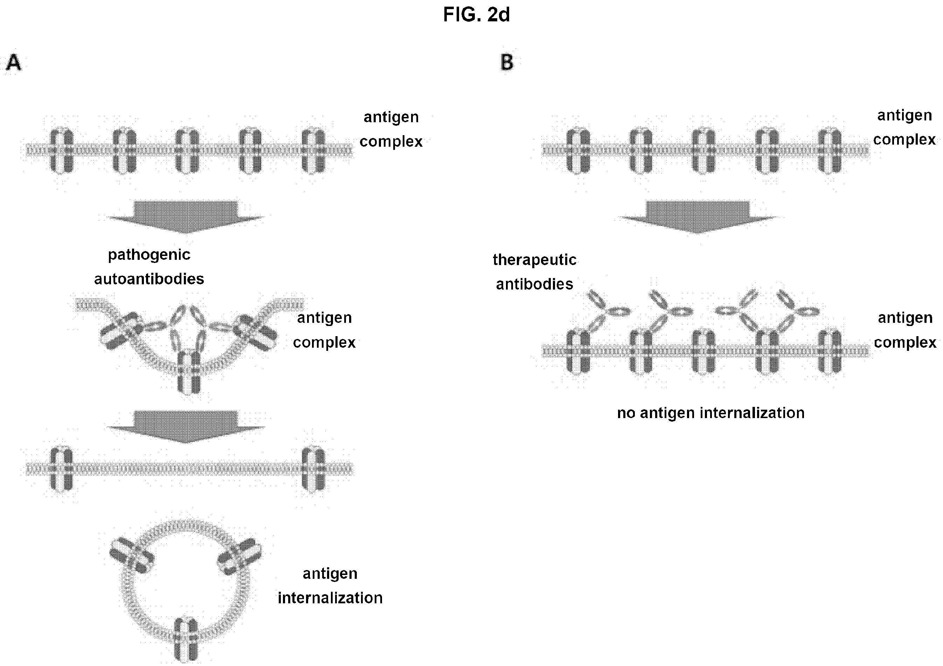

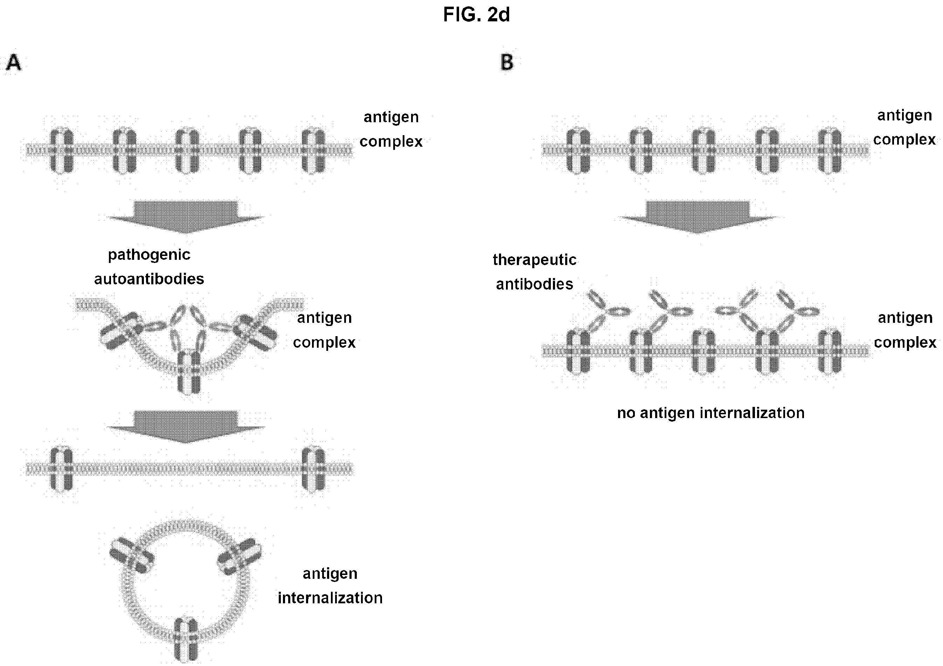

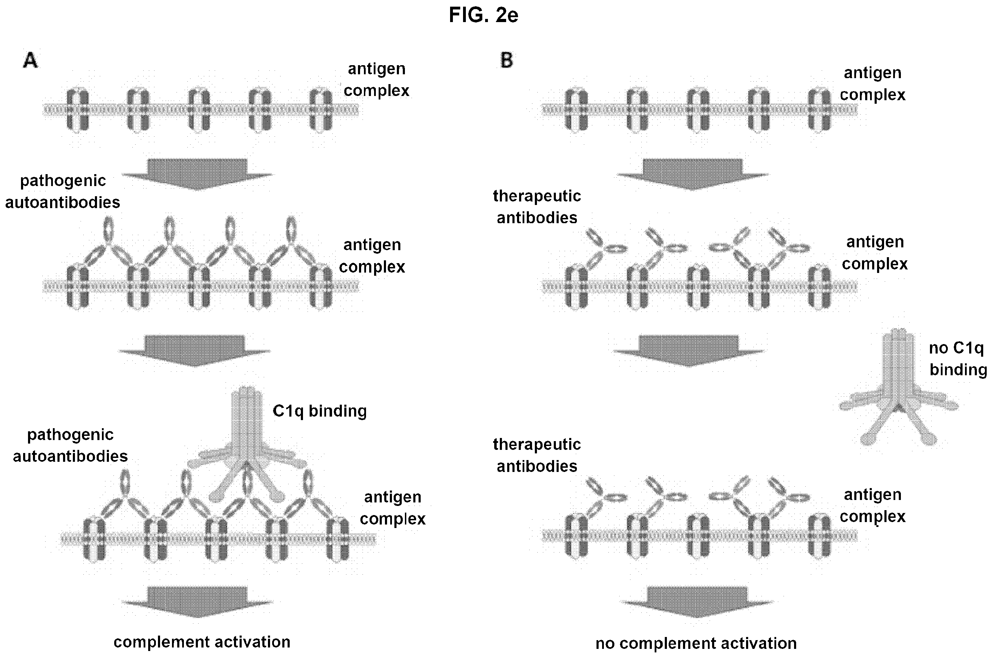

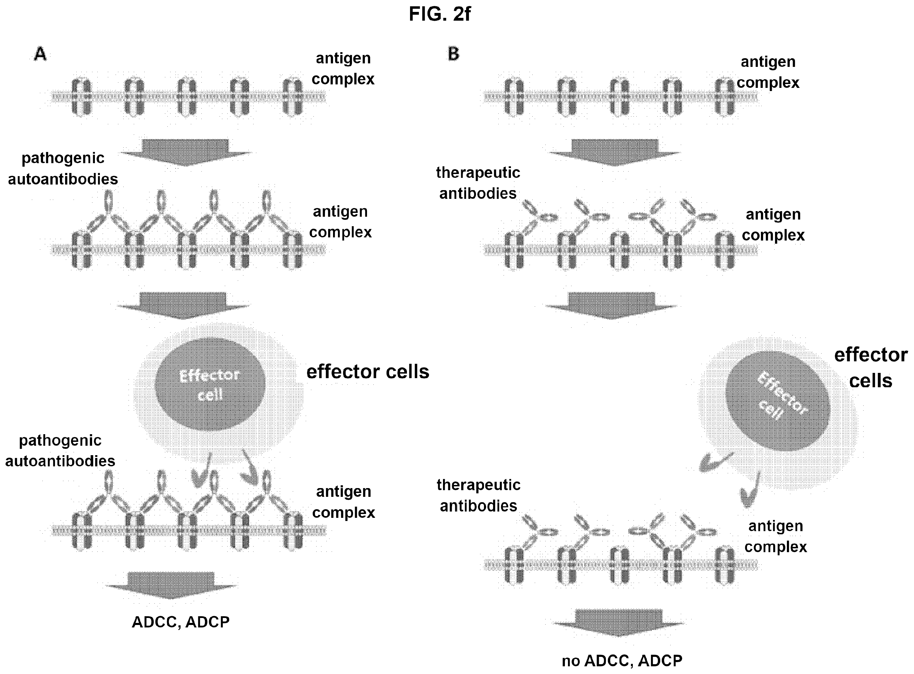

FIG. 2a is a schematic diagram illustrating the binding orientation of an autoantibody (A) or a therapeutic antibody to an autoantigen according to an embodiment of the present invention.
FIG. 2b is a schematic diagram showing differences occurring upon binding to an autoantigen depending on the difference between the binding orientation of the autoantibody (A) and the binding orientation of the therapeutic antibody (B) to the autoantigen according to the present invention.
FIG. 2c is a schematic diagram showing an antigen-antibody complex formed by cross-linking of the autoantibody with the autoantigen (A), and a schematic diagram illustrating a mechanism by which the therapeutic antibody inhibits the cross-linking between the autoantibody and the autoantigen (B).
FIG. 2d is a schematic diagram showing antigen internalization caused by the autoantibody (A), and a schematic diagram illustrating a mechanism by which the therapeutic antibody inhibits the internalization of the antigen (B).
FIG. 2e is a schematic diagram showing complement activation caused by the autoantibody (A), and a schematic diagram illustrating a mechanism for inhibiting complement-dependent cytotoxicity (B).
FIG. 2f is a schematic diagram showing antibody-dependent cellular cytotoxicity (ADCC) or antibody-dependent cellular phagocytosis (ADCP) caused by the autoantibody (A), and a schematic diagram illustrating a mechanism for inhibiting ADCC or ADCP by the therapeutic antibody (B).

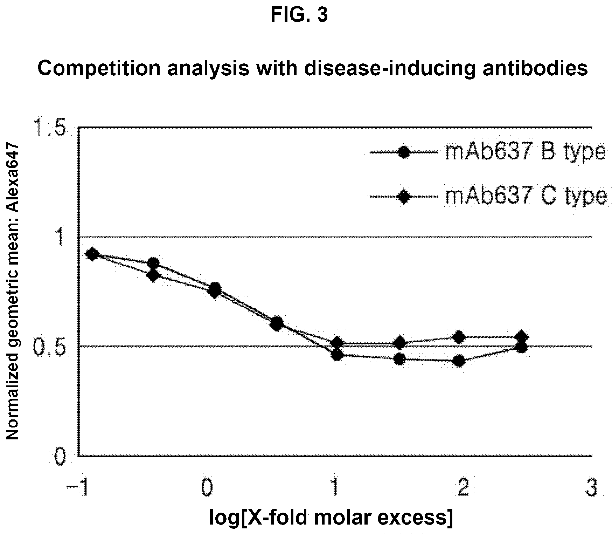

FIG. 3 is a graph showing the results of competitive binding analysis between the autoantibody mAb637 against the autoantigen AChR and recombinant antibodies mAb637 B and mAb637 C. The vertical axis represents the signal change according to the concentration of candidate antibodies in a normalized geometric mean Alexa647, and the horizontal axis represents the dilution factor in log scale (X-fold molar excess).

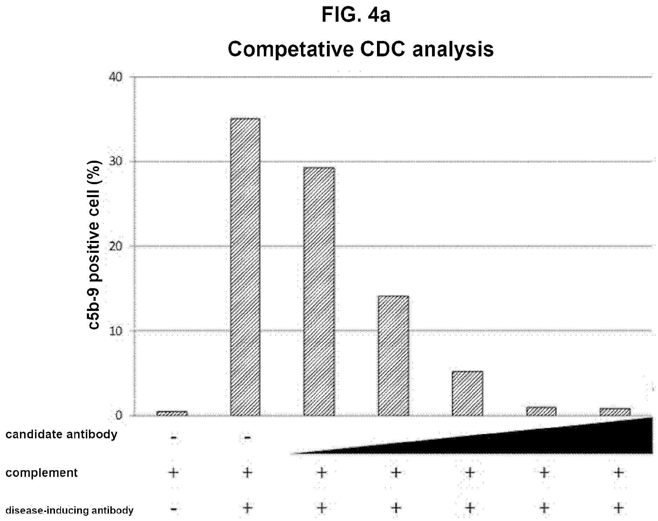

FIG. 4a is a graph analyzing the percentage of C5b-9 positive cells induced after contacting 293T cells-which express the AChR gene and have three complement inhibitor genes (CD46, CD55, CD59) knocked out-with autoantibody mAb35 alone, or with autoantibody mAb35 and the recombinant antibody of the present invention.

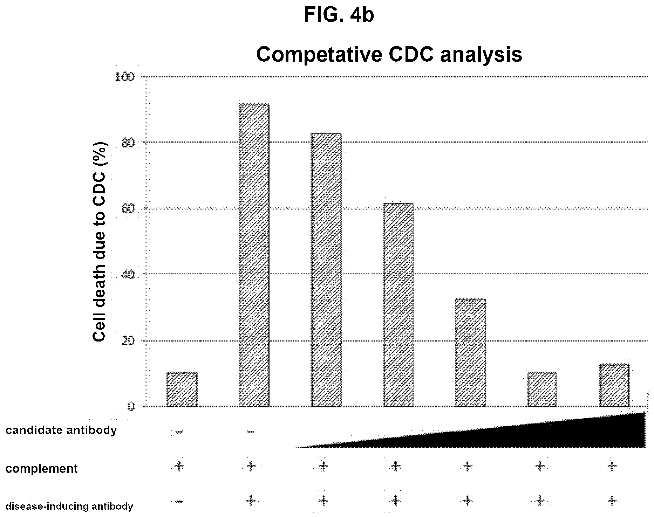

FIG. 4b is a graph showing the results of analyzing the cell death rate induced by CDC after contacting 293T cells-which express the AChR gene and have three complement inhibitor genes (CD46, CD55, CD59) knocked out-with autoantibody mAb35 alone, or with autoantibody mAb35 and the recombinant antibody of the present invention.

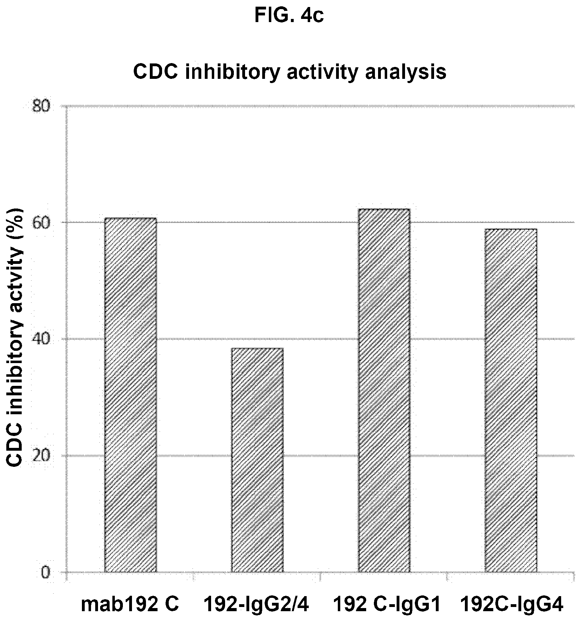

FIG. 4c is a graph showing the results of analyzing the level of inhibition of autoantibody-induced C5b-9 positive cells by competitive recombinant antibodies, following the contact of 293T cells (expressing the AChR gene and lacking three complement inhibitor genes) with the humanized mAb35 autoantibody and competitive recombinant antibody mAb192 C IgG2/4 and variants thereof.

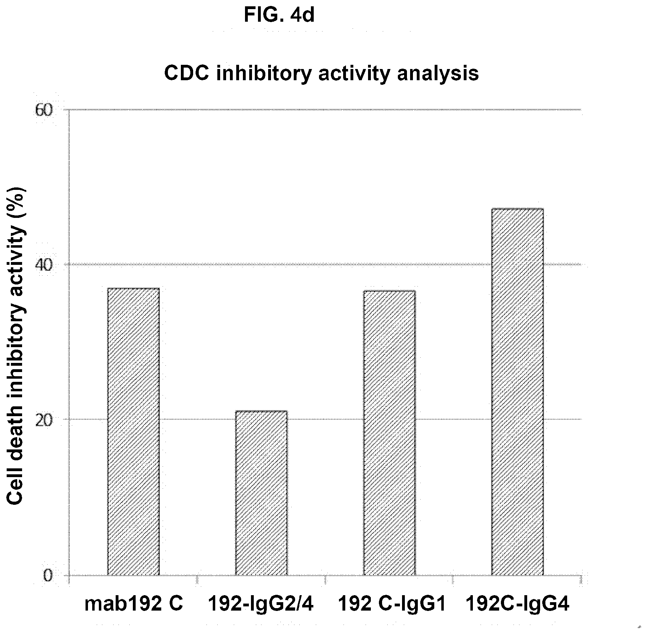

FIG. 4d is a graph showing the results of analyzing the level of inhibition of cell death induced by CDC by contacting 293T cells (expressing the AChR gene and lacking three complement inhibitor genes) with the humanized mAb35 autoantibody and competitive recombinant antibody mAb192 C IgG2/4 and variants thereof.

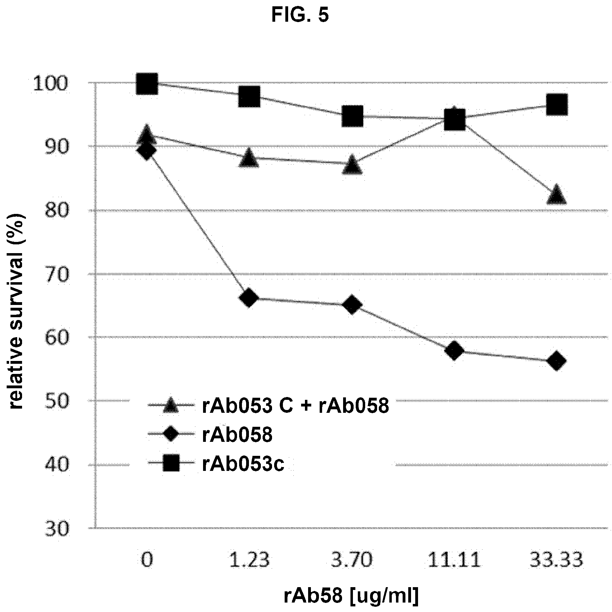

FIG. 5 is a graph showing the results of analyzing cell viability by contacting aquaporin-4-expressing U87MG cells with the NMO-pathogenic autoantibody rAb58 or the recombinant antibody rAb53 C, which competitively binds to the aquaporin-4 antigen.

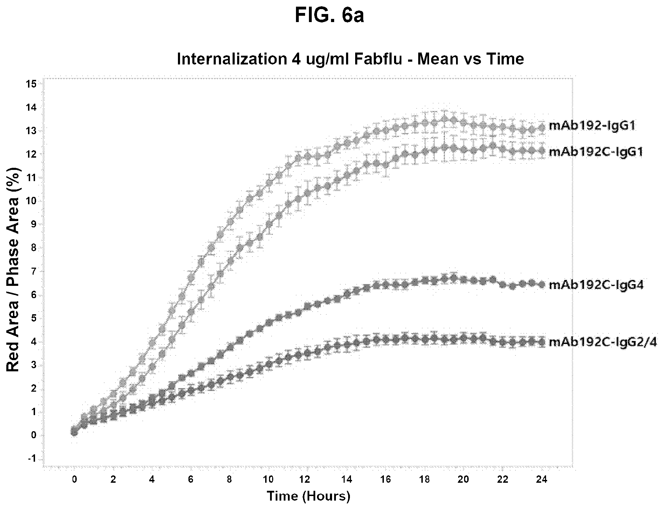

FIG. 6a is a graph showing the results of analyzing the pattern of AChR internalization into cells by contacting 293T cells (expressing the AChR gene and lacking three complement inhibitor genes) with autoantibody mAb192 or mAb192 variants. From the top, the graph shows the degree of internalization over time for mAb192, mAb192 C IgG1, mAb192 IgG2/4, mAb192 C IgG4, and mAb192 C IgG2/4.

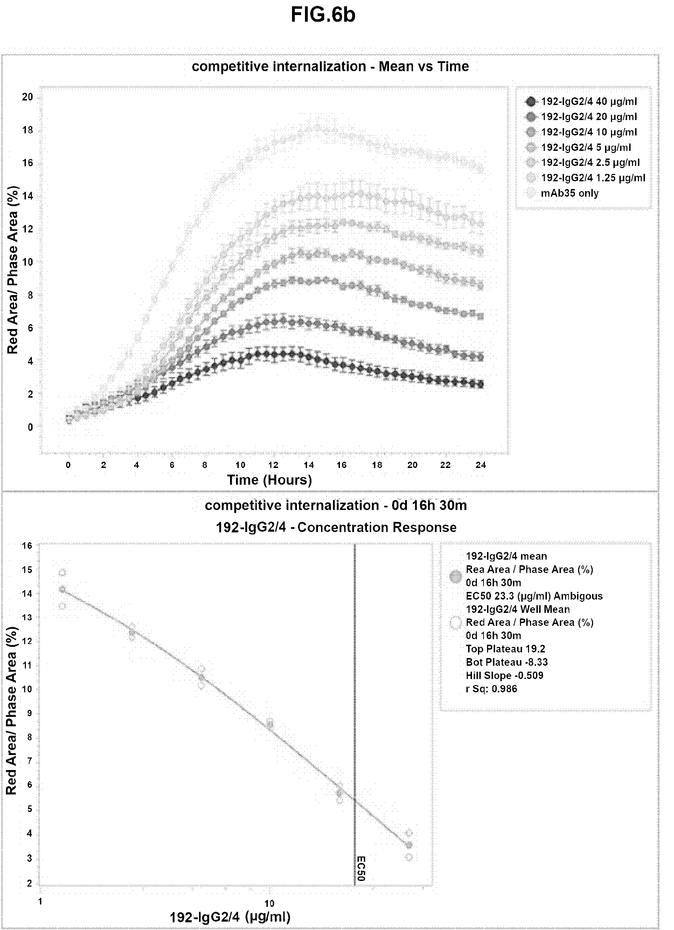

FIG. 6b is a graph showing the results of analyzing the pattern in which AChR internalization induced by the autoantibody mAb35 is inhibited by mAb192-IgG2/4 (a variant of mAb192), after contacting 293T cells (expressing the AChR gene and lacking three complement inhibitor genes) with mAb35 alone or with both mAb35 and mAb192 variants.

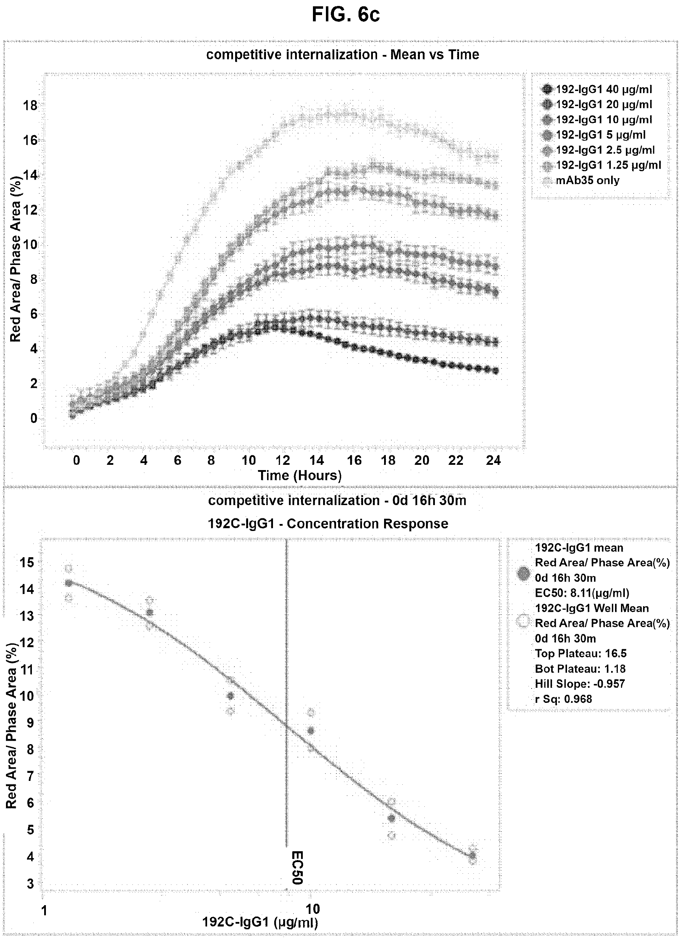

FIG. 6c is a graph showing the results of analyzing the pattern in which AChR internalization induced by the autoantibody mAb35 is inhibited by mAb192 C-IgG1 (a variant of mAb192), after contacting 293T cells (expressing the AChR gene and lacking three complement inhibitor genes) with mAb35 alone or with both mAb35 and mAb192 variants.

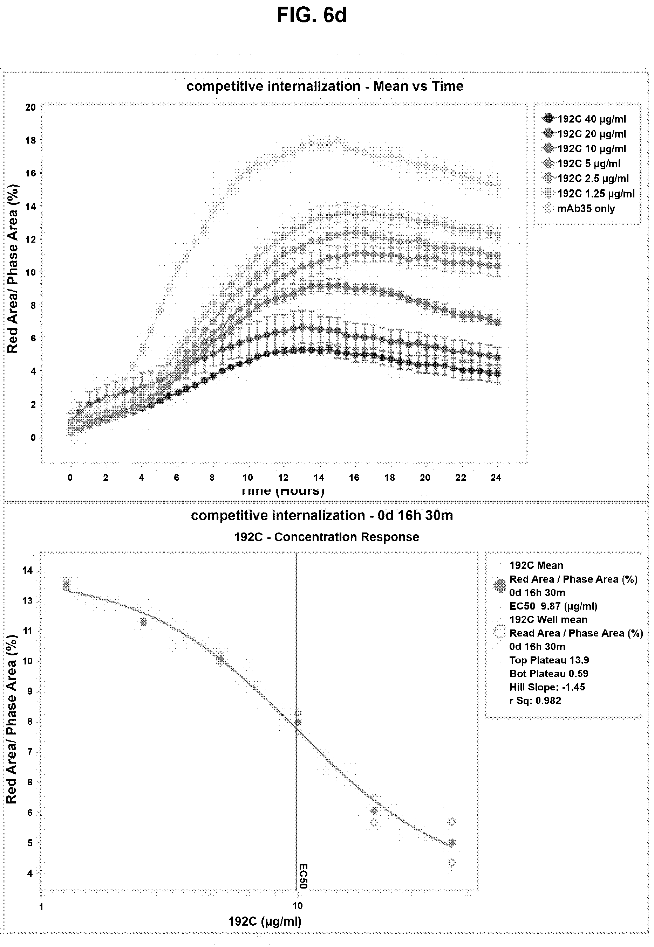

FIG. 6d is a graph showing the results of analyzing the pattern in which AChR internalization induced by the autoantibody mAb35 is inhibited by mAb192 C, after contacting 293T cells (expressing the AChR gene and lacking three complement inhibitor genes) with mAb35 alone or with both mAb35 and mAb192 variants.

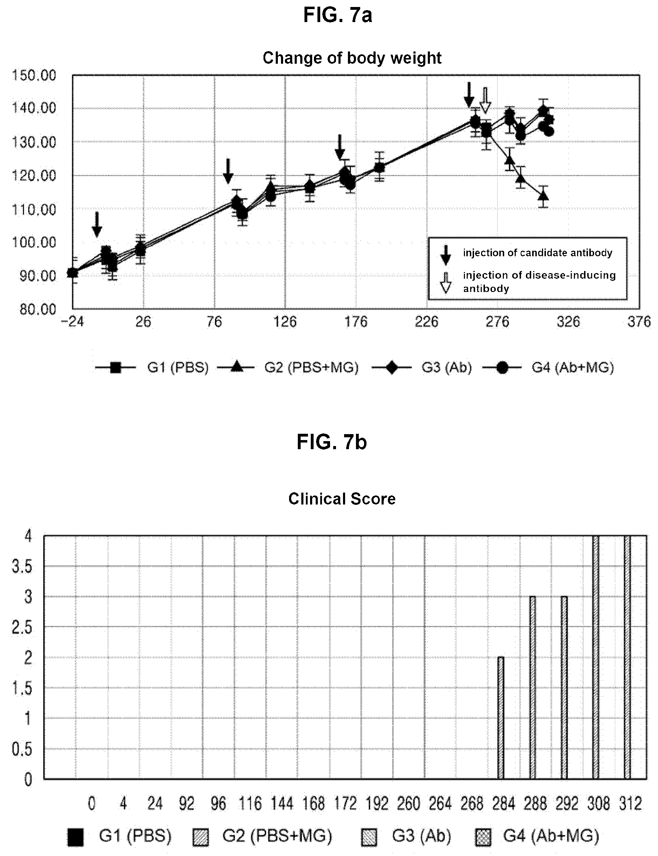

FIG. 7a is a graph showing the results of analyzing body weight changes over time in Lewis rats after repeatedly administering the therapeutic antibody mAb35 C (5 mg/kg, Ab) four times at 3.5-day intervals, followed by the administration of the myasthenia gravis-pathogenic autoantibody mAb35 (1.5 mg/kg, MG).
FIG. 7b is a graph showing the results of analyzing clinical score changes over time in Lewis rats after repeatedly administering the therapeutic antibody mAb35 C (5 mg/kg, Ab) four times at 3.5-day intervals, followed by the administration of the myasthenia gravis-pathogenic autoantibody mAb35 (1.5 mg/kg, MG).

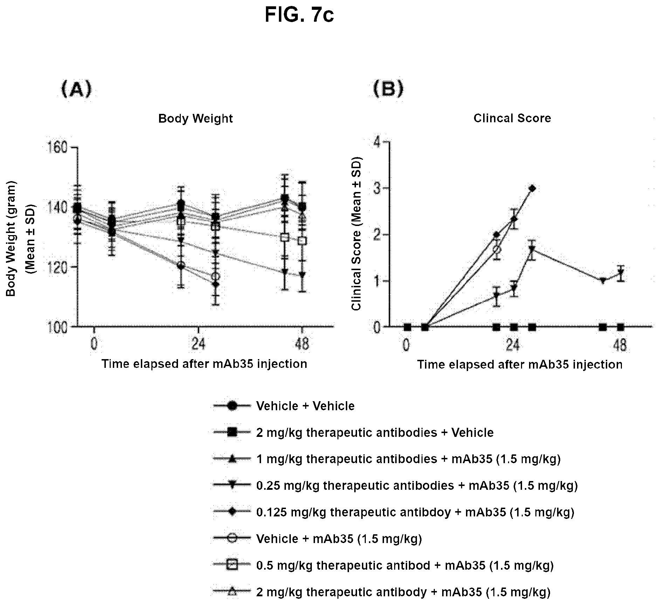

FIG. 7c is a graph showing the results of analyzing body weight changes (A) and clinical score changes (B) over time in Lewis rats after repeatedly administering the therapeutic antibody mAb35 C (0.125 to 2 mg/kg) four times at 3.5-day intervals, followed by the administration of the myasthenia gravis-pathogenic autoantibody mAb35 (1.5 mg/kg).

### Best Mode for Implementing the Invention

### Definition of Terms

Unless otherwise defined herein, technical and scientific terms used in this disclosure have the same meanings as commonly understood by one of ordinary skill in the art to which this disclosure belongs. For the purpose of understanding this disclosure, the following definitions will apply, and terms used in the singular include the plural unless the context clearly dictates otherwise, and vice versa. Terms such as first, second, etc., may be used to describe various components, but the components should not be limited by these terms. These terms are used only for the purpose of distinguishing one component from another. For example, a first component could be termed a second component, and similarly, a second component could be termed a first component, without departing from the scope of the present invention.

As used herein, the term "and/or" includes any and all combinations that the associated configurations can define.

As used herein, the terms "polypeptide" and "protein" may be used interchangeably to refer to a long-chain peptide having an amino acid sequence of a natural protein or an amino acid sequence having one or more mutations such as deletion, substitution, addition, and/or insertion of one or more amino acid residues.

As used herein, the term "antibody" includes a whole antibody, any antigen-binding fragment thereof, or a single chain thereof. The "antibody" may include a glycoprotein comprising at least two heavy chains (H) and two light chains (L) interconnected by disulfide bonds, or an antigen-binding portion thereof. Each heavy chain may comprise a heavy chain variable region (V_{H} or HV) and a heavy chain constant region. The heavy chain constant region may comprise four regions: CH1, hinge, CH2, and CH3. Each light chain may comprise a light chain variable region (V_{L} or LV) and a light chain constant region. The light chain constant region may consist of one region, CL. The V_{H} and V_{L} regions may be interspersed with more conserved regions called framework regions (FR) and subdivided into hypervariable regions (HVR) called complementarity determining regions (CDR). Each V_{H} and V_{L} may comprise three CDRs and four FRs arranged from amino-terminus to carboxy-terminus in the order of FR1, CDR1, FR2, CDR2, FR3, CDR3, and FR4. The variable regions of the heavy and light chains contain a binding domain that interacts with an antigen. The constant regions may mediate the binding of immunoglobulins to host tissues or factors, including various cells of the immune system (e.g., effector cells) and the first component (C1q) of the classical complement system.

As used herein, the term "half-antibody fragment" refers to an antigen-binding fragment comprising only one of the two antigen-binding sites of a whole antibody. The half-antibody fragment may comprise a light chain variable region (V_{L}) and a heavy chain variable region (V_{H}) and may have binding specificity for an autoantigen. The dimeric antibody of the present invention may comprise two half-antibody fragments. The half-antibody fragment may further comprise a light chain constant region and all or part of a heavy chain constant region. Specifically, it may further include a light chain constant region (CL) and any one or more of heavy chain constant regions CH1, CH2, and CH3. The half-antibody fragment may include V_{L}-CL and V_{H}-CH1, or V_{L}-CL and V_{H}-CH1-CH2-CH3. A boundary region between V_{L} and CL, between V_{H} and CH1, or between any of the domains of CH1-CH2-CH3 may include a linker. The linker may be, for example, an S-S sequence.

As used herein, the term "monoclonal antibody" refers to an antibody obtained from a population of substantially homogeneous antibodies, where individual antibodies within the population are identical and/or bind to the same epitope, except for possible minor variant antibodies. For example, monoclonal antibodies may be produced by various techniques, including hybridoma methods, recombinant DNA methods, phage-display methods, and methods using transgenic animals containing all or part of a human immunoglobulin locus.

As used herein, the term "binding affinity" refers to the intrinsic binding affinity reflecting a 1:1 interaction between members of a binding pair (e.g., an antibody and an antigen). The affinity of molecule X for its partner Y can generally be expressed by the dissociation constant (Kd). Affinity can be measured by conventional methods known in the art, including those described herein.

As used herein, the terms "F_{C}," "Fc site," and "Fc region" refer to a portion of an antibody molecule consisting of the hinge region or a part thereof, and the CH2 and CH3 regions. The Fc region of the IgG class, according to EU numbering (also called EU INDEX), refers to, for example, the region from the 226th cysteine to the C-terminus, or from the 230th proline to the C-terminus, but is not limited thereto. The Fc region can be appropriately obtained by partially digesting monoclonal antibodies such as IgG1, IgG2, IgG3, and IgG4 with proteases such as pepsin or papain, followed by re-eluting the fraction adsorbed onto a Protein A or Protein G column.

As used herein, the term "linker" refers to a nucleic acid, amino acid, or non-peptide residue that can be inserted between one or more molecules, for example, between one or more component regions. For example, a linker can be used to provide a desired site of interest between components to facilitate manipulation. Linkers may also be provided to enhance the expression of a fusion protein from a host cell and to reduce steric hindrance so that the components can assume their optimal tertiary structure and/or interact appropriately with target molecules. A linker sequence may include one or more amino acids naturally linked to a receptor component, or it may be an added sequence used to enhance expression, provide a site of interest, allow optimal tertiary structure formation, and/or enhance interaction with target molecules. Preferably, the linker increases the flexibility of the fusion protein components without interfering with the structure of each functional component within the fusion protein.

An "antibody that binds to the same epitope" as a reference antibody refers to an antibody that blocks binding of the reference antibody to its antigen by at least 10%, 20%, 30%, 40%, or 50% in a competition assay, and the reference antibody blocks binding of the antibody to its antigen by at least 10%, 20%, 30%, 40%, or 50%. For example, it may refer to an antibody that blocks binding of the reference antibody to its antigen by at least 50%, and vice versa.

As used herein, the term "human antibody" is an antibody possessing an amino acid sequence corresponding to that of an antibody produced by a human or human cell, or derived from a non-human source utilizing a human antibody repertoire or other human antibody-encoding sequences.

As used herein, the term "humanized antibody" refers to a molecule in which the antigen-binding portion of the molecule is substantially derived from immunoglobulins from a non-human species, while the remaining immunoglobulin structure of the molecule is based on the structure and/or sequence of human immunoglobulins.

As used herein, the term "chimeric antibody" refers to an antibody in which a portion of the heavy and/or light chain is derived from a particular source or species, while the remainder of the heavy and/or light chain is derived from a different source or species.

As used herein, the term "host cell" refers to a cell into which an exogenous nucleic acid has been introduced. Host cells include transformants and transformed cells, encompassing the primary transformed cell and progeny derived therefrom regardless of the number of passages.

As used herein, the term "pharmaceutical composition" refers to a formulation containing an active ingredient. Furthermore, the term "formulation" means that at least one additional component other than the active ingredient is present in the pharmaceutical composition. A pharmaceutical composition is a formulation suitable for administration to a subject, such as a human patient. It may be in a lyophilized form, for example, a solution formed after reconstitution of a lyophilized pharmaceutical composition using saline or water, or in the form of a solution that does not require reconstitution. The pharmaceutical composition may be liquid or solid.

As used herein, the term "administration" or "administering" refers to the step of providing a pharmaceutical composition or an active ingredient to a subject. Pharmaceutical compositions can be administered through various appropriate routes.

As used herein, the term "therapeutically effective amount" refers to a level, amount, or concentration of a pharmaceutical composition comprising an agent required to treat a disease, disorder, or condition without causing significant negative or adverse side effects.

As used herein, the terms "treat," "treating," or "treatment" refer to the alleviation or reduction (including partial, substantial, near-complete, and complete reduction), resolution, or prevention (temporary or permanent) of a disease, disorder, or condition to achieve a desired therapeutic result-for example, by healing injured or damaged tissue, or by altering, changing, enhancing, improving, ameliorating, and/or beautifying an existing or perceived disease, disorder, or condition. "Prevention" refers to the delay of the onset of a disease, disorder, or condition. Prevention may be considered complete if the onset is delayed for a predetermined period of time.

As used herein, the term "combination" refers to any form of two or more different therapeutic agents such that a second therapeutic agent is administered while a previously administered therapeutic agent is still effective in the body. For example, two therapeutic agents are effective in a subject simultaneously, which may include a synergistic effect of the two agents. Different therapeutic agents may be administered simultaneously or sequentially as a single formulation or separate formulations.

According to one aspect of the present invention, there is provided a recombinant homodimeric antibody having specificity for an autoantigen, which is derived from an autoantibody having specificity for the autoantigen and comprises a light chain and a heavy chain,

wherein the heavy chain comprises at least a portion of a light chain variable region (V_{L}) of the autoantibody, and the light chain comprises at least a portion of a heavy chain variable region (V_{H}) of the autoantibody. For example, some regions of the variable regions of the heavy chain and light chain of the autoantibody may be exchanged with each other.

The autoantibody may have pathogenicity for an autoimmune disease.

The antibody of the present invention in one embodiment may have variable regions (e.g., complementarity determining regions) derived from the autoantibody. That is, the antibody of one embodiment may comprise an amino acid sequence corresponding to the variable regions of the autoantibody. For example, the heavy chain variable region of the antibody of one embodiment may be derived from the light chain variable region of the autoantibody, and the light chain variable region may be derived from the heavy chain variable region of the autoantibody. When the antibody of an embodiment comprises a constant region, the constant region may be derived from the autoantibody, in which case the sequence of the constant region of the autoantibody may be preserved as is, or may further include amino acid mutations (e.g., amino acid substitution, deletion, and/or insertion). It goes without saying that the constant region in the antibody of an embodiment may not be derived from the autoantibody.

Among individual functional domains of the present antibody, domains derived from the autoantibody may have a homology of at least 99%, at least 98%, at least 95%, or at least 90% with the corresponding amino acid sequence of the autoantibody. In this case, the activity (e.g., binding affinity to the autoantigen) of the mutated antibody of the present invention may be at least 50%, at least 60%, at least 70%, at least 80%, at least 90%, or at least 95% identical to the activity of the non-mutated homodimeric antibody.

In the homodimeric antibody, the autoantigen may be a membrane protein; a plurality of the autoantigens may be cross-linked with each other by an autoantibody to form an antigen-antibody complex structure; and the antigen-antibody complex may be internalized by the cross-linking.

In the homodimeric antibody, a complement binding may be induced by the cross-linking; and the homodimeric antibody may bind to the same epitope as the epitope of the autoantigen to which the autoantibody binds, or to a site adjacent to the same epitope, thereby inhibiting the binding of the autoantibody to the autoantigen.

That is, the homodimeric antibody may reduce or prevent the formation of a cross-linking structure through the binding of a plurality of the autoantigens with the autoantibody. Furthermore, the homodimeric antibody may reduce or prevent the internalization of the antigen-antibody complex caused by the cross-linking.

In the homodimeric antibody, a binding orientation of at least one monomer of the homodimeric antibody to the autoantigen may be different from a binding orientation of the autoantibody to the autoantigen; and the binding of one monomer of the homodimeric antibody to a first autoantigen may cause the remaining monomer of the homodimeric antibody to have a binding orientation that prevents it from binding to a second autoantigen present on the cell surface.

In the homodimeric antibody, it may have a conformation that does not bind to a plurality of complex autoantigens among complex autoantigens present on the cell surface; it may not cross-link two or more complex autoantigens present on the cell surface; and an Fc region of the heavy chain may be an IgG1 Fc, an IgG4 Fc, or an IgG2/4 hybrid Fc.

In the homodimeric antibody, the autoantibody comprises a first half-antibody fragment comprising a first light chain variable region (V_{L}) and a first heavy chain variable region (V_{H}) and having specificity for an autoantigen, and a second half-antibody fragment comprising a second light chain variable region (V_{L}) and a second heavy chain variable region (V_{H}) and having specificity for the autoantigen;

wheirein the homodimeric antibody may comprise at least one of the following half-antibody fragments:
i) a heavy chain comprising the first light chain variable region (V_{L}), and a light chain comprising the first heavy chain variable region (V_{H});
ii) a heavy chain comprising the second light chain variable region (V_{L}), and a light chain comprising the second heavy chain variable region (V_{H});
iii) a heavy chain comprising at least one of CDRL1, CDRL2, and CDRL3 of the first light chain variable region (V_{L}), and a light chain comprising at least one of CDRH1, CDRH2, and CDRH3 of the first heavy chain variable region (V_{H}) and the remaining CDRL portions not included in the heavy chain; and
iv) a heavy chain comprising at least one of CDRL1, CDRL2, and CDRL3 of the second light chain variable region (V_{L}), and a light chain comprising at least one of CDRH1, CDRH2, and CDRH3 of the second heavy chain variable region (V_{H}) and the remaining CDRL portions not included in the heavy chain.

In the homodimeric antibody, the autoantibody may comprise a first half-antibody fragment comprising a first light chain variable region and a first heavy chain variable region and having specificity for an autoantigen, and a second half-antibody fragment comprising a second light chain variable region and a second heavy chain variable region and having specificity for the autoantigen

The homodimeric antibody may be one in which V_{L}-CL and V_{H}-CH1 of at least one half-antibody fragment among the half-antibody fragments of the autoantibody are exchanged with each other.

According to another aspect of the present invention, there is provided a pharmaceutical composition for preventing or treating autoimmune diseases, comprising the homodimeric antibody and a pharmaceutically acceptable carrier.

According to another aspect of the present invention, there is provided a recombinant dimeric antibody comprising a light chain variable region and a heavy chain variable region and having specificity for an autoantigen,

wherein in at least one monomer of the recombinant dimeric antibody, at least a portion of a light chain variable region (V_{L}) and at least a portion of a heavy chain variable region (V_{H}) of an autoantibody-which has specificity for the autoantigen and has pathogenicity for an autoimmune disease-are exchanged with each other.

According to another aspect of the present invention, there is provided a method for treating autoimmune diseases, comprising the step of administering the pharmaceutical composition to a subject suffering from an autoimmune disease.

According to another aspect of the present invention, there is provided a dimeric antibody comprising a light chain variable region and a heavy chain variable region and having specificity for an autoantigen. The dimeric antibody may be a recombinant dimeric antibody. The dimeric antibody may be a heterodimeric antibody or a homodimeric antibody. For example, the dimeric antibody may be a homodimeric antibody. In the dimeric antibody, at least a portion of a light chain variable region and at least a portion of a heavy chain variable region of an autoantibody having specificity for the autoantigen may be exchanged with each other. The autoantibody may have specificity for the autoantigen and pathogenicity for an autoimmune disease. When the dimeric antibody is a heterodimeric antibody, one monomer may be one in which at least a portion of the light chain variable region and at least a portion of the heavy chain variable region of the autoantibody are exchanged, while the other monomer may be one in which the amino acid sequence of the autoantibody is preserved. Alternatively, in the heterodimeric antibody, both monomers may have exchanged light chain variable regions and heavy chain variable regions, but the exchanged regions may be different.

Unless contradictory herein, descriptions of a heterodimeric antibody may be equally applied to a homodimeric antibody, and vice versa. In the present disclosure, the homodimeric antibody may be one in which both monomers have exchanged regions between at least a portion of the light chain variable region and at least a portion of the heavy chain variable region of the autoantibody, wherein the exchanged regions are identical.

The dimeric antibody may comprise two monomers. For example, the dimeric antibody may comprise a first half-antibody fragment comprising a first light chain variable region and a first heavy chain variable region, and a second half-antibody fragment comprising a second light chain variable region and a second heavy chain variable region. In one embodiment, at least one monomer of the dimeric antibody (e.g., the first half-antibody fragment and/or the second half-antibody fragment) may be one in which at least a portion of the light chain variable region and at least a portion of the heavy chain variable region of the autoantibody that binds to the autoantigen are exchanged with each other. That is, the first and/or second light chain variable regions of the dimeric antibody may be sequences corresponding to at least a portion of the heavy chain variable region of the autoantibody, and the first and/or second heavy chain variable regions of the dimeric antibody may be sequences corresponding to at least a portion of the light chain variable region of the autoantibody. The autoantibody may have specificity for the autoantigen and pathogenicity for an autoimmune disease.

In an embodiment of the present invention, when the dimeric antibody is a heterodimeric antibody, one of the monomers (e.g., the first or second half-antibody fragment) may be one in which at least a portion of the light chain variable region and at least a portion of the heavy chain variable region of the autoantibody having specificity for the autoantigen are exchanged with each other.

The 'dimeric antibody' of one embodiment binds to an autoantigen and competitively inhibits an autoantibody, as will be described later, and may be referred to herein by terms such as "competitive autoantibody inhibitor', 'candidate antibody', or "therapeutic antibody'.

The autoantibody having specificity for the autoantigen may be naturally found in a subject. The subject may be a mammal including a human. The autoantibody may be a pathogenic autoantibody for an autoimmune disease. For example, the autoantibody may be a pathogenic autoantibody for an autoimmune disease associated with a reduction in autoantigen function. For example, the autoantibody may be a pathogenic autoantibody that induces autoimmune disease by binding to aquaporin-4 (an autoantigen) and causing antigen internalization, ADCC induction, CDC induction, and/or reduction of ADCP. For example, the autoantibody may be a pathogenic autoantibody for myasthenia gravis and/or neuromyelitis optica. The autoantibody may be a whole antibody. The autoantibody may comprise a light chain variable region-light chain constant region (V_{L}-CL) and a heavy chain variable region-heavy chain constant region 1-heavy chain constant region 2-heavy chain constant region 3 (V_{H}-CH1-CH2-CH3). The autoantibody may have two antigen-binding sites. Each antigen-binding site of the autoantibody may bind to the same epitope.

The autoantibody may bind to the autoantigen. The autoantibody may be a dimeric antibody, and each fragment of the autoantibody may bind to the same epitope of the autoantigen. Thus, the autoantibody may be a bivalent antibody. For example, the autoantibody may be a monospecific bivalent antibody.

The dimeric antibody of the present invention may bind to the autoantigen. The first half-antibody fragment or the second half-antibody fragment may each bind to the same epitope of the autoantigen. The competitive autoantibody inhibitor may be a bivalent antibody. For example, the competitive autoantibody inhibitor may be a monospecific bivalent antibody.

The autoantibody may bind to a complex autoantigen. The complex autoantigen may be an autoantigen comprising a plurality of subunit proteins. The complex autoantigen comprises a plurality of subunit proteins, and the subunit proteins may include two or more identical subunits. For example, the complex autoantigen may be an acetylcholine receptor (AChR) comprising five subunits, including two alpha subunits. The complex autoantigen may be an integral transmembrane protein. The complex autoantigen may have an epitope on the cell surface. The epitope may be, for example, the main immunogenic region (MIR) of the alpha subunit of AChR. The autoantibody may be, for example, monoclonal antibody IgG1-637 that binds to the main immunogenic region (MIR) of the alpha subunit of human AChR. The antibody binding site in the MIR may be residues 1-32 or 60-81. The autoantibody that binds to the alpha subunit of AChR may induce myasthenia gravis.

Furthermore, the complex autoantigen may be aquaporin-4 (AQP4). Neuromyelitis optica (NMO) can develop when autoantibodies against aquaporin-4, a water channel in neurons, are produced. Aquaporin-4 exists as isoforms called M1 or M23. Among them, M23 forms clusters and has increased binding affinity for autoantibodies. Therefore, the M23 form can be considered pathogenic.

In patients with neuromyelitis optica, loss of aquaporin-4 in the central nervous system is observed; the aquaporin-4 antibody is found in more than 95% of neuromyelitis optica patients and is an autoantibody having CDC or NK-based cytotoxicity. Removal of aquaporin-4 antibodies from plasma improves the symptoms of neuromyelitis optica, and transferring aquaporin-4 antibodies to healthy animals results in symptoms similar to neuromyelitis optica. Aquaporin-4 may have a tetrameric structure. The complex autoantigen may have an epitope on the cell surface. Residues 148-149 and 151 of extracellular loop C, residues 226 and 228 of loop E, residues 63-65 and 69 of extracellular loop A, residues 141, 151, and 154 of loop C, and/or residues 230-231 of loop E of aquaporin-4 may be important residues for binding with pathogenic autoantibodies. For example, the epitope of aquaporin-4 that binds with pathogenic autoantibodies may include residues 148-149 and 151 of extracellular loop C, residues 226 and 228 of loop E, residues 63-65 and 69 of extracellular loop A, residues 141, 151, and 154 of loop C, and/or residues 230-231 of loop E. A non-limiting example of the autoantibody is rAb53, which is a ratderived sequence that binds to aquaporin-4. The two aforementioned major isoforms of aquaporin-4 are expressed in astrocytes. The long isoform (M1) initiates translation at Met-1, and the short isoform (M23) initiates translation at Met-23. M23 aquaporin-4 is assembled in the membrane as a regular square array called orthogonal arrays of particles (OAP) when expressed. OAP formation by M23 results from tetramer-tetramer interactions involving residues just downstream of Met-23 at the cytoplasmic N-terminus, whereas residues just upstream of Met-23 in M1 disrupt this interaction. M1 does not form OAPs on its own but co-assembles with M23 to form heterotetramers that limit OAP size. A characteristic of neuromyelitis optica (NMO) is the presence of autoantibodies against aquaporin-4 (NMO-IgG) in the serum. The presence of NMO-IgG is specific for neuromyelitis optica, and according to some studies, serum NMO-IgG titers correlate with NMO disease activity. NMO-IgG is pathogenic for neuromyelitis optica. NMO-IgG may bind to the extracellular domain of M1 or M23, and is particularly known to have high affinity for M23.

Hereinafter, various structures of dimeric antibodies included in the antibody platform of the present invention and their various uses for preventing or treating autoimmune diseases will be exemplarily described with reference to the drawings.

FIG. 1 is a schematic diagram illustrating the structure of an antibody according to an embodiment. FIG. 1A illustrates an autoantibody against an autoantigen, and FIGS. 1B to 1D exemplarily illustrate antibodies according to an embodiment. Although a homodimeric antibody structure is exemplarily illustrated in FIG. 1, it goes without saying that the embodiments are not limited thereto.

As described above, in one embodiment, at least one monomer or both monomers of the antibody may comprise at least a portion of the light chain variable region and at least a portion of the heavy chain variable region of the autoantibody that binds to the autoantigen. The antibody may be one in which some regions of the variable regions of the heavy chain and light chain of the autoantibody are exchanged with each other.

In the antibody of one embodiment, the exchange may be an exchange of one or more, two or more, or three or more contiguous amino acid residues, or an exchange of functional domains. The exchange may result in a conformation different from the conformation of the complex obtained by the binding of the autoantigen and the autoantibody, while maintaining binding affinity for the antigen. The different conformation may be such that when one antigen-binding site binds to a complex autoantigen present on the cell surface, the other antigen-binding site has an orientation that prevents it from binding to another complex autoantigen.

In the antibody of one embodiment, the exchanged one or more, two or more, or three or more contiguous amino acid residues, or functional domains may comprise additional mutations. For example, the sequence of the exchanged one or more, two or more, or three or more contiguous amino acid residues, or functional domains may have at least 99% homology, 98% homology, 97% homology, 96% homology, 95% homology, or 90% homology with the sequence of the contiguous amino acid residues or functional domains of the autoantibody, and at the same time, the binding affinity for the antigen may be maintained at 50% or more, 60% or more, 70% or more, 80% or more, 90% or more, or 95% or more.

Referring to FIG. 1, the antibody of one embodiment may include: an antibody in which the light chain variable region (V_{L}) and light chain constant region (C_{L}) and the heavy chain variable region (V_{H}) and heavy chain constant region 1 (CH1) of the autoantibody are exchanged with each other (FIG. 1B); an antibody in which the light chain variable region (V_{L}) and the heavy chain variable region (V_{H}) of the autoantibody are exchanged with each other (FIG. 1C); or an antibody in which one or more of CDRL1, CDRL2, and CDRL3 of the light chain variable region (V_{L}) and one or more of CDRH1, CDRH2, and CDRH3 of the heavy chain variable region (V_{H}) are exchanged with each other (FIG. 1D). In the case of the antibody shown in FIG. 1D, CDRL1, CDRL2, and CDRL3 of the light chain variable region (V_{L}) and CDRH1, CDRH2, and CDRH3 of the heavy chain variable region (V_{H}) may be exchanged.

Furthermore, although not shown, each monomer of the dimeric antibody may have different regions exchanged. For example, one monomer of the dimeric antibody may be one in which the V_{L} and CL and the V_{H} and CH1 of the autoantibody are exchanged as in FIG. 1B, while the other monomer may be an antibody in which the V_{L} and the V_{H} are exchanged as in FIG. 1C.

In addition, although FIG. 1 illustrates that the aforementioned exchange occurs in both monomers of the antibody of one embodiment, such exchange may occur in only one of the monomers of the dimer.

In the dimeric antibody of one embodiment, the two monomers may be referred to as a first half-antibody fragment and a second half-antibody fragment, respectively. The first half-antibody fragment may further comprise a first light chain constant region (CL) and a first heavy chain constant region (CH), and the second half-antibody fragment may further comprise a second light chain constant region (CL) and a second heavy chain constant region (CH). The first heavy chain constant region (CH) and the second heavy chain constant region (CH) may each comprise heavy chain constant region 1 (CH1), heavy chain constant region 2 (CH2), and heavy chain constant region 3 (CH3). The antibody of one embodiment may include an antibody in which V_{L}-CL and V_{H}-CH1 are exchanged in the first half-antibody fragment, an antibody in which V_{L}-CL and V_{H}-CH1 are exchanged in the second half-antibody fragment, or an antibody in which V_{L}-CL and V_{H}-CH1 are exchanged in both the first and second half-antibody fragments.

The autoantigen may be a membrane protein. The autoantigen may be an intrinsic membrane protein or an extrinsic membrane protein, and specifically, it may be a transmembrane protein or an integral monotopic protein (e.g., an integral monotopic protein attached to the outside of the cell membrane). The monotopic protein may interact with one layer of the phospholipid bilayer. For example, the epitope of the autoantigen for the autoantibody may be distributed on the cell surface. The autoantigen may be a complex autoantigen comprising a plurality of subunit proteins. The complex autoantigen comprises a plurality of subunit proteins, and the subunit proteins may comprise two or more identical subunits.

The autoantibody may bind to the identical subunit of a first complex antigen and bind to the identical subunit of a second complex antigen, thereby cross-linking the first complex antigen and the second complex antigen. The complex of the autoantibody and the complex antigen may be internalized by the cross-linking. The identical subunit may be, for example, the alpha subunit of AChR, and the autoantibody may bind to the MIR epitope of the alpha subunit.

Complement binding may be induced by the cross-linking. The complement may bind to the Fc region of the antibody bound to the first complex autoantigen and the second complex autoantigen. Antibody-dependent cellular cytotoxicity (ADCC) and complement-dependent cytotoxicity (CDC) may be induced by the binding.

The antibody of one embodiment may not cause such problems by not binding to a plurality of autoantigens. This will be exemplarily described below with reference to FIGS. 2a to 2f.

FIGS. 2a to 2f are schematic diagrams illustrating the therapeutic mechanism of immune diseases caused by autoantibodies, showing the change in binding orientation between the antibody of one embodiment and an autoantigen and the resulting therapeutic mechanism.

FIG. 2a is a schematic diagram showing the binding orientation of an antibody (FIG. 2a-B) or an autoantibody (FIG. 2a-A) with an autoantigen according to an embodiment.

FIG. 2b is a schematic diagram showing the difference in binding modes between an antibody and an autoantigen resulting from the difference in binding orientation between the antibody and the autoantigen (FIG. 2b-B) and the autoantibody and the autoantigen (FIG. 2b-A) according to an embodiment.

FIG. 2c is a schematic diagram showing a mechanism by which the antibody according to an embodiment inhibits the formation of a cross-linked structure by the binding of an autoantigen and an autoantibody.

FIG. 2d is a schematic diagram showing a mechanism by which the antibody according to an embodiment inhibits internalization of an antigen.

FIG. 2e is a schematic diagram showing a mechanism by which the antibody according to an embodiment inhibits complement-dependent cytotoxicity caused by an autoantibody.

FIG. 2f is a schematic diagram showing a mechanism by which the antibody according to an embodiment inhibits antibody-dependent cellular cytotoxicity or antibody-dependent cellular phagocytosis.

Referring to FIGS. 2a and 2b, in the antibody of one embodiment, the binding orientation of at least one monomer among the dimers of the dimeric antibody to the autoantigen may be different from the binding orientation of the autoantibody to the autoantigen. Accordingly, the binding of one monomer of the dimer to a first autoantigen may have an orientation that prevents the remaining monomer of the dimer from binding to a second autoantigen (the same autoantigen as the first autoantigen) present on the cell surface. For example, the binding orientation of the first half-antibody fragment or the second half-antibody fragment to the autoantigen may be different from the binding orientation of the autoantibody to the autoantigen.

Therefore, the first half-antibody fragment and/or the second half-antibody fragment may bind to the same epitope as the epitope to which the autoantibody fragment binds, or to an epitope at a position adjacent to the same epitope, thereby inhibiting the binding of the autoantibody to the autoantigen. For example, the first and second half-antibody fragments may bind to the same epitope as the autoantibody fragment. Since the binding orientation of the first half-antibody fragment (or the second half-antibody fragment) to the first autoantigen is different from that of the autoantibody, the second half-antibody fragment (or the first half-antibody fragment) that is not bound to the autoantigen may be unable to bind to the second autoantigen present on the cell surface.

That is, the antibody of one embodiment may have a conformation that makes it difficult to bind or prevents it from binding to a plurality (e.g., two) of complex autoantigens among complex autoantigens present on the cell surface.

Referring to FIG. 2c, the antibody of one embodiment can inhibit the cross-linking of a plurality of autoantibodies through the binding of autoantibodies to autoantigens. For example, the antibody of one embodiment may be one that does not cross-link a plurality (e.g., 2, 3, 4, 5 or more) of complex autoantigens present on the cell surface. Since the antibody of one embodiment can inhibit the formation of a cross-linked structure by the binding of autoantibodies and autoantigens even with a small amount of administration, it can exhibit preventive or therapeutic effects at a low dosage. That is, the antibody or competitive autoantibody inhibitor of one embodiment may have a low effective dose.

Referring to FIG. 2d, it can be confirmed that even if the antibody of one embodiment binds to a complex autoantigen present on the cell surface, antigen internalization is not induced because a plurality of autoantigens are not cross-linked. Therefore, the antibody of one embodiment can prevent or treat immune diseases caused by immune responses against cells where antigens are present as the antigens are internalized, or by the loss of antigen function.

Since the antibody of one embodiment can prevent autoantigens from being cross-linked and internalized, it can exhibit excellent preventive or therapeutic effects on diseases associated with autoantigens formed densely on cells.

Referring to FIGS. 2e and 2f, unlike the autoantibody, the antibody of one embodiment does not form a cross-linked structure by binding to autoantigens. Therefore, complement activation is suppressed because complements such as C1q cannot bind to the antibody. In addition, since the antibody of one embodiment cannot bind to FcγR and inhibits antigen binding of the autoantibody, it can suppress the occurrence of ADCC and/or CDC through FcγR. Therefore, even if the competitive autoantibody inhibitor binds to a complex autoantigen present on the cell surface, ADCC and/or CDC may not be induced. Thus, it is effective for preventing or treating autoimmune diseases (e.g., myasthenia gravis and/or neuromyelitis optica) in which CDC and/or ADCC are major pathogenic factors.

The antibody of one embodiment may lack the effector functions of an intact antibody. For example, mutations may be introduced into the Fc region to reduce binding of the antibody to activating Fcγ receptors (FcγR) and to reduce Fc effector functions such as C1q binding, complement-dependent cytotoxicity (CDC), antibody-dependent cell-mediated cytotoxicity (ADCC), or antibody-dependent phagocytosis (ADCP). Fc positions that can be mutated to reduce antibody binding to activating FcγR and subsequently reduce effector functions include positions 214, 233, 234, 235, 236, 237, 238, 265, 267, 268, 270, 295, 297, 309, 327, 328, 329, 330, 331, and 365. Exemplary mutations that can be made singly or in combination are mutations K214T, E233P, L234V, L234A, deletion of G236, V234A, F234A, L235A, G237A, P238A, P238S, D265A, S267E, H268A, H268Q, Q268A, N297A, A327Q, P329A, D270A, Q295A, V309L, A327S, L328F, A330S, and P331S of IgG1, IgG2, IgG3, or IgG4. Exemplary combination mutations resulting in antibodies with reduced ADCC include mutations L234A/L235A on IgG1, V234A/G237A/P238S/H268A/V309L/A330S/P331S on IgG2, F234A/L235A on IgG4, S228P/F234A/L235A on IgG4, N297A on all Ig isotypes, V234A/G237A on IgG2, K214T/E233P/L234V/L235A/G236-deletion/A327G/P331A/D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, S228P/F234A/L235A/G237A/P238S on IgG4, and S228P/F234A/L235A/G236-deletion/G237A/P238S on IgG4. Hybrid IgG2/4 Fc domains, such as an Fc having residues 117-260 from IgG2 and residues 261-447 from IgG4, may also be used. An exemplary mutation resulting in an antibody with reduced CDC is the K322A mutation. To improve IgG4 stability, the well-known S228P mutation can be made in IgG4 antibodies.

The antibody may comprise a mutated Fc region lacking effector functions, such as an IgG1 sequence comprising L234A/L235A amino acid substitutions, L234A/L235A/G237A amino acid substitutions, and/or K322A amino acid substitutions in the Fc region. In addition, the inhibitor may include IgG2 or IgG3 having substitutions in the Fc region that eliminate effector functions such as complement activation. Alternatively, the inhibitor may be an IgG4 antibody. The inhibitor may be optimized for binding to an antigen, for example, AChR or aquaporin-4, by mutating some residues in the antigen-binding region. The antibody may be humanized.

The antibody may comprise mutations in the Fc region that adjust half-life. Fc positions that can be mutated to adjust antibody half-life (e.g., binding to FcRn) include positions 250, 252, 253, 254, 256, 257, 307, 376, 380, 428, 434, and 435. Exemplary mutations that can be made singly or in combination are mutations T250Q, M252Y, I253A, S254T, T256E, P257I, T307A, D376V, E380A, M428L, H433K, N434S, N434A, N434H, N434F, H435A, and H435R. Exemplary single or combination mutations that can be prepared to reduce the half-life of an antibody are mutations H435A, P257I/N434H, D376V/N434H, M252Y/S254T/T256E/H433K/N434F, T308P/N434A, and H435R.

Hereinafter, the antibody according to one embodiment will be described with specific examples. Although the antibodies described below in one embodiment may be animal-derived antibodies, the present invention may include humanized antibodies thereof.

The antibodies may be mAb637 C and mAb637 B, which competitively bind to the myasthenia gravis autoantibody mAb637-IgG1 and the AChR antigen. mAb637 C comprises a heavy chain of SEQ ID NO: 19 and a light chain of SEQ ID NO: 20, and mAb637 B comprises a heavy chain of SEQ ID NO: 17 and a light chain of SEQ ID NO: 18, respectively.

The antibodies may be mAb192 C IgG2/4 and mAb192 B, which competitively bind to the myasthenia gravis autoantibody mAb637-IgG1 and the AChR antigen. mAb192 C IgG2/4 comprises a heavy chain of SEQ ID NO: 3 and a light chain of SEQ ID NO: 4. mAb192 B comprises a heavy chain of SEQ ID NO: 5 and a light chain of SEQ ID NO: 6, respectively. In addition, the antibodies may be competitive inhibitory recombinant antibodies mAb35 B and mAb35 C of mAb35, which competitively bind to the myasthenia gravis pathogenic autoantibody mAb35 and the AChR antigen. The recombinant antibody mAb35 B comprises a heavy chain of SEQ ID NO: 13 and a light chain of SEQ ID NO: 14, and the recombinant antibody mAb35 C comprises a heavy chain of SEQ ID NO: 15 and a light chain of SEQ ID NO: 16. Also, the antibody may be rAb53 C, which competitively binds to the neuromyelitis optica pathogenic autoantibody rAb58 and the aquaporin-4 antigen. rAb53 C comprises a heavy chain of SEQ ID NO: 9 and a light chain of SEQ ID NO: 10. The rAb53 autoantibody may have a heavy chain of SEQ ID NO: 47 and a light chain of SEQ ID NO: 48.

The antibodies may be mAb637 C and mAb637 B, which competitively bind to the myasthenia gravis autoantibody mAb637-IgG1 and the AChR antigen. mAb637 C and mAb637 B comprise complementarity-determining regions of SEQ ID NOs: 21, 22, 23, 24, 25, and 26.

The V_{L} of mAb637 B and mAb637 C comprises light chain CDR1 of SEQ ID NO: 21, light chain CDR2 of SEQ ID NO: 22, and light chain CDR3 of SEQ ID NO: 23, and the V_{H} comprises heavy chain CDR1 of SEQ ID NO: 24, heavy chain CDR2 (EDN) of SEQ ID NO: 25, and heavy chain CDR3 of SEQ ID NO: 26.

The antibodies may be mAb192 C IgG2/4 and mAb192 B, which competitively bind to the myasthenia gravis autoantibody mAb637-IgG1 and the AChR antigen. mAb192 C IgG2/4 and mAb192 B comprise complementarity-determining regions of SEQ ID NOs: 27, 28, 29, 30, 31, and 32.

The V_{L} of mAb192 B and mAb192 C IgG2/4 comprises light chain CDR1 of SEQ ID NO: 27, light chain CDR2 of SEQ ID NO: 28, and light chain CDR3 of SEQ ID NO: 29, and the V_{H} may comprise heavy chain CDR1 of SEQ ID NO: 30, heavy chain CDR2 of SEQ ID NO: 31, and heavy chain CDR3 of SEQ ID NO: 32. In addition, the antibodies may be competitive inhibitory recombinant antibodies mAb35 B and mAb35 C of mAb35, which competitively bind to the myasthenia gravis pathogenic autoantibody mAb35 and the AChR antigen. The recombinant antibodies mAb35 B and mAb35 C comprise complementarity-determining regions of SEQ ID NOs: 33, 34, 35, 36, 37, and 38.

The V_{L} of mAb35 B and mAb35 C comprises light chain CDR1 of SEQ ID NO: 33, light chain CDR2 of SEQ ID NO: 34, and light chain CDR3 of SEQ ID NO: 35, and the V_{H} may comprise heavy chain CDR1 of SEQ ID NO: 36, heavy chain CDR2 (KTN) of SEQ ID NO: 37, and heavy chain CDR3 of SEQ ID NO: 38.

Also, the antibody may be rAb53 C, which competitively binds to the neuromyelitis optica pathogenic autoantibody rAb58 and the aquaporin-4 antigen. rAb53 C comprises complementarity-determining regions of SEQ ID NOs: 39, 40, 41, 42, 43, and 44. The rAb53 autoantibody may have a heavy chain of SEQ ID NO: 47 and a light chain of SEQ ID NO: 48.

The V_{L} of rAb53 C comprises light chain CDR1 of SEQ ID NO: 39, light chain CDR2 of SEQ ID NO: 40, and light chain CDR3 of SEQ ID NO: 41, and the V_{H} may comprise heavy chain CDR1 of SEQ ID NO: 42, heavy chain CDR2 (GAS) of SEQ ID NO: 43, and heavy chain CDR3 of SEQ ID NO: 44.

According to another aspect of the present invention, there is provided a pharmaceutical composition for use in preventing or treating a disease associated with a reduction in autoantigen function, comprising the competitive autoantibody inhibitor as an active ingredient and a pharmaceutically acceptable carrier.

A pharmaceutically acceptable carrier enhances or stabilizes the composition or facilitates the preparation of the composition. The carrier includes physiologically compatible solvents, dispersion media, coatings, antibacterial and antifungal agents, isotonic and absorption-delaying agents, and the like.

The composition of the present invention may be administered by various methods known in the relevant technical field. The route and/or mode of administration vary depending on the desired result. Administration may be intravenous, intramuscular, intraperitoneal, or subcutaneous, or may be administered adjacent to a target site. The carrier may be suitable for intravenous, intramuscular, subcutaneous, parenteral, spinal, or epidermal administration (for example, by injection or infusion). The composition may be sterile and fluid. The composition may be in a lyophilized form. The composition may include an isotonic agent, for example, a sugar, a polyalcohol such as mannitol or sorbitol, and sodium chloride. The composition of the present invention may be prepared according to methods widely known and commonly practiced in the relevant technical field.

The dosage level of the active ingredient in the composition may be varied to obtain an amount of the active ingredient effective to achieve the desired therapeutic response for a particular patient, composition, and mode of administration without being toxic to the patient. The selected dosage level depends on various pharmacokinetic factors, such as the specific composition of the present disclosure used, or the route of administration, time of administration, rate of excretion, duration of treatment, other drugs, compounds and/or materials used in combination, the age, sex, weight, condition, general health, and prior medical history of the patient being treated, and other factors. As a non-limiting example, the dosage ranges from approximately 0.0001 to 100 mg/kg of body weight, and more typically from 0.1 to 20 mg/kg of body weight. An exemplary treatment regimen involves administration once every week, or once every two weeks, or once every month, or once every 3 to 6 months.

In the pharmaceutical composition of the present invention, the disease may be an autoimmune disease. The autoimmune disease may be pemphigus (pemphigus vulgaris, pemphigus foliaceus, or paraneoplastic pemphigus), Crohn's disease, idiopathic thrombocytopenic purpura (ITP), heparin-induced thrombocytopenia (HIT), thrombotic thrombocytopenic purpura (TTP), myasthenia gravis (MG), and chronic inflammatory demyelinating polyneuropathy (CIDP). Additional non-limiting autoimmune diseases include autoimmune thrombocytopenia, immune neutropenia, anti-hemophilic FVIII inhibitors, antiphospholipid syndrome, Kawasaki syndrome, ANCA-associated disease, polymyositis, bullous pemphigoid, multiple sclerosis (MS), Guillain-Barré syndrome, chronic polyneuropathy, ulcerative colitis, diabetes mellitus, autoimmune thyroiditis, Graves' ophthalmopathy, rheumatoid arthritis, ulcerative colitis, primary sclerosing cholangitis, systemic lupus erythematosus (SLE), autoimmune encephalomyelitis, Hashimoto's thyroiditis, Goodpasture syndrome, autoimmune hemolytic anemia, scleroderma with anti-collagen antibodies, mixed connective tissue disease, pernicious anemia, idiopathic Addison's disease, autoimmune-related infertility, glomerulonephritis (e.g., crescentic glomerulonephritis, proliferative glomerulonephritis), insulin resistance, and autoimmune diabetes (type 1 diabetes, insulin-dependent diabetes). Autoimmune diseases have also been recognized to include atherosclerosis and Alzheimer's disease. In another embodiment, the autoimmune disease may be selected from the group consisting of hepatitis, autoimmune hemophilia, autoimmune lymphoproliferative syndrome (ALPS), autoimmune uveitis, glomerulonephritis, agammaglobulinemia, alopecia areata, amyloidosis, ankylosing spondylitis, autoimmune angioedema, autoimmune aplastic anemia, autoimmune dysautonomia, autoimmune hyperlipidemia, autoimmune immunodeficiency, autoimmune inner ear disease (AIED), autoimmune myocarditis, autoimmune pancreatitis, autoimmune retinopathy, autoimmune urticaria, autoimmune urticarial neuropathy, autoimmune axonal neuropathy, Baló's disease, Behcet's disease, Castleman's disease, celiac disease, Chagas disease, chronic recurrent multifocal osteomyelitis (CRMO), Churg-Strauss syndrome, cicatricial pemphigoid, benign mucosal pemphigoid, Cogan's syndrome, cold agglutinin disease, Coxsackie myocarditis, CREST disease, essential mixed cryoglobulinemia, dermatitis herpetiformis, dermatomyositis, Devic's disease (neuromyelitis optica), dilated cardiomyopathy, discoid lupus, Dressler's syndrome, endometriosis, eosinophilic angiocentric fibrosis, eosinophilic fasciitis, erythema nodosum, Evans syndrome, fibrosing alveolitis, giant cell arteritis (temporal arteritis), Hashimoto's encephalitis, Henoch-Schönlein purpura, herpes gestationis, idiopathic hypocomplementemic tubulointerstitial nephritis, multiple myeloma, multifocal motor neuropathy, NMDA receptor antibody encephalitis, IgG4-related disease, IgG4-related sclerosing disease, inflammatory aortic aneurysm, inflammatory pseudotumor, inclusion body myositis, interstitial cystitis, juvenile arthritis, Kuttner tumor, Lambert-Eaton syndrome, leukocytoclastic vasculitis, lichen planus, lichen sclerosus, ligneous conjunctivitis, linear IgA disease (LAD), Lyme disease, chronic, mediastinal fibrosis, Meniere's disease, microscopic polyangiitis, Mikulicz's syndrome, Mooren's ulcer, Mucha-Habermann disease, multifocal fibrosclerosis, narcolepsy, optic neuritis, Ormond's disease (retroperitoneal fibrosis), palindromic rheumatism, PANDAS (pediatric autoimmune neuropsychiatric disorders associated with streptococcus), paraneoplastic cerebellar degeneration, paraproteinemic polyneuropathy, paroxysmal nocturnal hemoglobinuria (PNH), Parry-Romberg syndrome, Parsonage-Turner syndrome, arthritis, periarteritis, peripheral neuropathy, perivenous encephalomyelitis, POEMS syndrome, polyarteritis nodosa, type I, II, & III autoimmune polyglandular syndromes, polymyalgia rheumatica, postpericardiotomy syndrome, progesterone dermatitis, primary biliary cirrhosis, psoriasis, psoriatic arthritis, idiopathic pulmonary fibrosis, pyoderma gangrenosum, pure red cell aplasia, Raynaud's phenomenon, reflex sympathetic dystrophy, Reiter's syndrome, relapsing polychondritis, restless legs syndrome, rheumatic fever, Riedel's thyroiditis, sarcoidosis, Schmidt syndrome, scleritis, Sjögren's syndrome, sperm and testicular autoimmunity, stiff-person syndrome, subacute bacterial endocarditis (SBE), Susac's syndrome, sympathetic ophthalmia, Takayasu's arteritis, Tolosa-Hunt syndrome, transverse myelitis, undifferentiated connective tissue disease (UCTD), vesiculobullous dermatosis, vitiligo, Rasmussen's encephalitis, and Waldenström's macroglobulinemia.

For example, the disease may be selected from the group consisting of pemphigus vulgaris, pemphigus foliaceus, paraneoplastic pemphigus, rheumatoid arthritis, systemic lupus erythematosus, Crohn's disease, idiopathic thrombocytopenic purpura (ITP), heparin-induced thrombocytopenia (HIT), thrombotic thrombocytopenic purpura (TTP), autoimmune hemolytic anemia (AIHA), myasthenia gravis (MG), chronic inflammatory demyelinating polyneuropathy (CIDP), multifocal motor neuropathy, neuromyelitis optica, autoimmune thrombocytopenia, immune neutropenia, anti-hemophilic FVIII inhibitors, antiphospholipid syndrome, Kawasaki syndrome, ANCA-associated disease, polymyositis, dermatomyositis, bullous pemphigoid, multiple sclerosis (MS), Guillain-Barré syndrome, chronic polyneuropathy, ulcerative colitis, diabetes mellitus, autoimmune thyroiditis, Graves' ophthalmopathy, autoimmune urticaria, vasculitis, and Rasmussen's encephalitis.

According to another aspect of the present invention, there is provided a method for preventing or treating a disease associated with a decrease in the function of an autoantigen in a subject, comprising administering to the subject an amount of the antibody effective to prevent or treat the disease.

According to another aspect of the present invention, there is provided the use of the antibody for the prevention or treatment of a disease associated with a decrease in the function of an autoantigen.

According to another aspect of the present invention, there is provided the use of the antibody in the manufacture of a medicament for the prevention or treatment of a disease associated with a decrease in the function of an autoantigen.

In the present specification, all descriptions regarding the aforementioned antibody can be equally applied to the pharmaceutical composition, the method for prevention or treatment, the use for prevention or treatment, and the use in the manufacture of a medicament for prevention or treatment.

In the method, the disease may be an autoimmune disease, and the disease may be pemphigus (pemphigus vulgaris, pemphigus foliaceus, or paraneoplastic pemphigus), Crohn's disease, idiopathic thrombocytopenic purpura (ITP), heparin-induced thrombocytopenia (HIT), thrombotic thrombocytopenic purpura (TTP), myasthenia gravis (MG), and chronic inflammatory demyelinating polyneuropathy (CIDP). Additional non-limiting autoimmune diseases include autoimmune thrombocytopenia, immune neutropenia, anti-hemophilic FVIII inhibitors, antiphospholipid syndrome, Kawasaki syndrome, ANCA-associated disease, polymyositis, bullous pemphigoid, multiple sclerosis (MS), Guillain-Barré syndrome, chronic polyneuropathy, ulcerative colitis, diabetes mellitus, autoimmune thyroiditis, Graves' ophthalmopathy, rheumatoid arthritis, ulcerative colitis, primary sclerosing cholangitis, systemic lupus erythematosus (SLE), autoimmune encephalomyelitis, Hashimoto's thyroiditis, Goodpasture syndrome, autoimmune hemolytic anemia, scleroderma with anti-collagen antibodies, mixed connective tissue disease, pernicious anemia, idiopathic Addison's disease, autoimmune-related infertility, glomerulonephritis (e.g., crescentic glomerulonephritis, proliferative glomerulonephritis), insulin resistance, and autoimmune diabetes (type 1 diabetes, insulin-dependent diabetes). Autoimmune diseases have also been recognized to include atherosclerosis and Alzheimer's disease. In another embodiment, the autoimmune disease may be selected from the group consisting of hepatitis, autoimmune hemophilia, autoimmune lymphoproliferative syndrome (ALPS), autoimmune uveitis, glomerulonephritis, agammaglobulinemia, alopecia areata, amyloidosis, ankylosing spondylitis, autoimmune angioedema, autoimmune aplastic anemia, autoimmune dysautonomia, autoimmune hyperlipidemia, autoimmune immunodeficiency, autoimmune inner ear disease (AIED), autoimmune myocarditis, autoimmune pancreatitis, autoimmune retinopathy, autoimmune urticaria, autoimmune urticarial neuropathy, autoimmune axonal neuropathy, Baló's disease, Behcet's disease, Castleman's disease, celiac disease, Chagas disease, chronic recurrent multifocal osteomyelitis (CRMO), Churg-Strauss syndrome, cicatricial pemphigoid, benign mucosal pemphigoid, Cogan's syndrome, cold agglutinin disease, Coxsackie myocarditis, CREST disease, essential mixed cryoglobulinemia, dermatitis herpetiformis, dermatomyositis, Devic's disease (neuromyelitis optica), dilated cardiomyopathy, discoid lupus, Dressler's syndrome, endometriosis, eosinophilic angiocentric fibrosis, eosinophilic fasciitis, erythema nodosum, Evans syndrome, fibrosing alveolitis, giant cell arteritis (temporal arteritis), Hashimoto's encephalitis, Henoch-Schönlein purpura, herpes gestationis, idiopathic hypocomplementemic tubulointerstitial nephritis, multiple myeloma, multifocal motor neuropathy, NMDA receptor antibody encephalitis, IgG4-related disease, IgG4-related sclerosing disease, inflammatory aortic aneurysm, inflammatory pseudotumor, inclusion body myositis, interstitial cystitis, juvenile arthritis, Kuttner tumor, Lambert-Eaton syndrome, leukocytoclastic vasculitis, lichen planus, lichen sclerosus, ligneous conjunctivitis, linear IgA disease (LAD), Lyme disease, chronic, mediastinal fibrosis, Meniere's disease, microscopic polyangiitis, Mikulicz's syndrome, Mooren's ulcer, Mucha-Habermann disease, multifocal fibrosclerosis, narcolepsy, optic neuritis, Ormond's disease (retroperitoneal fibrosis), palindromic rheumatism, PANDAS (pediatric autoimmune neuropsychiatric disorders associated with streptococcus), paraneoplastic cerebellar degeneration, paraproteinemic polyneuropathy, paroxysmal nocturnal hemoglobinuria (PNH), Parry-Romberg syndrome, Parsonage-Turner syndrome, arthritis, periarteritis, peripheral neuropathy, perivenous encephalomyelitis, POEMS syndrome, polyarteritis nodosa, type I, II, & III autoimmune polyglandular syndromes, polymyalgia rheumatica, postpericardiotomy syndrome, progesterone dermatitis, primary biliary cirrhosis, psoriasis, psoriatic arthritis, idiopathic pulmonary fibrosis, pyoderma gangrenosum, pure red cell aplasia, Raynaud's phenomenon, reflex sympathetic dystrophy, Reiter's syndrome, relapsing polychondritis, restless legs syndrome, rheumatic fever, Riedel's thyroiditis, sarcoidosis, Schmidt syndrome, scleritis, Sjögren's syndrome, sperm and testicular autoimmunity, stiff-person syndrome, subacute bacterial endocarditis (SBE), Susac's syndrome, sympathetic ophthalmia, Takayasu's arteritis, Tolosa-Hunt syndrome, transverse myelitis, undifferentiated connective tissue disease (UCTD), vesiculobullous dermatosis, vitiligo, Rasmussen's encephalitis, and Waldenström's macroglobulinemia.

For example, the disease may be selected from the group consisting of pemphigus vulgaris, pemphigus foliaceus, paraneoplastic pemphigus, rheumatoid arthritis, systemic lupus erythematosus, Crohn's disease, idiopathic thrombocytopenic purpura (ITP), heparin-induced thrombocytopenia (HIT), thrombotic thrombocytopenic purpura (TTP), autoimmune hemolytic anemia (AIHA), myasthenia gravis (MG), chronic inflammatory demyelinating polyneuropathy (CIDP), multifocal motor neuropathy, neuromyelitis optica, autoimmune thrombocytopenia, immune neutropenia, anti-hemophilic FVIII inhibitors, antiphospholipid syndrome, Kawasaki syndrome, ANCA-associated disease, polymyositis, dermatomyositis, bullous pemphigoid, multiple sclerosis (MS), Guillain-Barré syndrome, chronic polyneuropathy, ulcerative colitis, diabetes mellitus, autoimmune thyroiditis, Graves' ophthalmopathy, autoimmune urticaria, vasculitis, and Rasmussen's encephalitis.

The subject may be an animal, the animal may be a mammal, and the mammal may be a human, dog, cat, cow, pig, monkey, mouse, or horse.

The administration can be performed by various methods known in the relevant technical field. The route and/or mode of administration vary depending on the desired result. Administration may be intravenous, intramuscular, intraperitoneal, or subcutaneous, or may be administered adjacent to a target site. The administration may be the administration of a composition containing the aforementioned antibody. A pharmaceutically acceptable carrier may be suitable for intravenous, intramuscular, subcutaneous, parenteral, spinal, or epidermal administration (for example, by injection or infusion). The composition may be sterile and fluid. The composition may be in a lyophilized form. Isotonic agents, for example, sugar, sugar alcohols such as mannitol or sorbitol, and sodium chloride may be included in the composition.

In the method, the dosage level of the active ingredient may be varied to obtain an amount of the active ingredient effective to achieve the desired therapeutic response for a particular patient, composition, and mode of administration without being toxic to the patient. The selected dosage level depends on various pharmacokinetic factors, such as the specific active ingredient or composition including the same of the present disclosure used, or the route of administration, time of administration, rate of excretion, duration of treatment, other drugs, compounds and/or materials used in combination, the age, sex, weight, condition, general health, and prior medical history of the patient, and other factors. As a non-limiting example, the dosage ranges from approximately 0.0001 to 100 mg/kg of body weight, and more typically from 0.1 to 20 mg/kg of body weight. An exemplary treatment regimen may involve administration once every week, or once every two weeks, or once every month, or once every 3 to 6 months.

According to another aspect of the present invention, there is provided the use of the antibody in the manufacture of a pharmaceutical composition for preventing or treating a disease associated with a decrease in the function of an autoantigen.

Hereinafter, the present invention will be described in more detail through examples. However, these examples are intended to illustratively explain the present invention, and the scope of the present invention is not limited to these examples.

### Example 1: Competitive Autoantibodies and Their Efficacy

### 1-1: Autoantibody mAb637 and its Competitive Autoantibody Inhibitors

The monoclonal antibody mAb637 was used as an antibody that induces Myasthenia Gravis (MG) pathology in humans. The mAb637 antibody is an AChR-specific monoclonal autoantibody isolated from a human MG patient. The mAb637 antibody, also referred to as IgG1-637, belongs to the G1 class and possesses effector functions. This antibody binds to the Main Immunogenic Region (MIR) of the AChR alpha subunit. In this experiment, the mAb637 antibody was produced in-house by cloning the DNA based on the published sequence, followed by transient expression in ExpiCHO cells and purification using MabSelect SuRe, a column used for purifying or isolating biopharmaceuticals. Passive transfer of the mAb637 antibody into a subject induces MG. mAb637 comprises a heavy chain having the amino acid sequence of SEQ ID NO: 1 and a light chain having the amino acid sequence of SEQ ID NO: 2. The V_{H} and V_{L} of mAb637 comprise the amino acid sequences of residues 1 to 126 of SEQ ID NO: 1 and residues 1 to 112 of SEQ ID NO: 2, respectively.

### 1-2: Recombinant Antibodies According to an Embodiment for Competitively Inhibiting MG Autoantibodies for in vitro CDC and Competition Assays

mAb637 C: This antibody has a V_{L}-SS-CH1-hinge-CH2-CH3(IgG2/4) + V_{H}-kappa structure. Specifically, the structure of mAb637 C was designed by exchanging the V_{L} and V_{H} from the light chain (SEQ ID NO: 2) of the V_{L}-CL structure and the heavy chain (SEQ ID NO: 1) of the V_{H}-CH1-hinge-CH2-CH3 structure of mAb637 (a human-derived MG-inducing antibody). Consequently, in the heavy chain where V_{H} was substituted with V_{L}, the junction between V_{L} and CH1-hinge-CH2-CH3 was connected via an -S-S- linker, and the CH1, CH2, and CH3 regions were converted from IgG1 to IgG2/4. Here, the CL is a kappa chain. The heavy and light chains of mAb637 C have the amino acid sequences of SEQ ID NO: 19 and SEQ ID NO: 20, respectively.

mAb637 B: This antibody has a V_{L}-kappa-hinge-CH2+CH3(IgG2/4) + V_{H}-CH1(IgG2/4) structure.

Specifically, mAb637 B was prepared as follows: First, in the light chain of the V_{L}-CL structure (SEQ ID NO: 2) and the heavy chain of the V_{H}-CH1-hinge-CH2-CH3 structure (SEQ ID NO: 1) of mAb637, which is a human-derived myasthenia gravis-inducing antibody, the V_{L}-CL and V_{H}-CH1 were exchanged. In the resulting heavy chain of V_{L}-CL-hinge-CH2-CH3 and the light chain of V_{H}-CH1, the CH1 and CH2-CH3 regions were modified to the IgG2/4 type, respectively. The heavy and light chains of mAb637 B prepared in this manner have the amino acid sequences of SEQ ID NO: 17 and SEQ ID NO: 18, respectively.

mAb192 C IgG2/4: This antibody has a V_{L}-SS-IgG2/4 + V_{H}-kappa structure. In the V_{L}-CL light chain (SEQ ID NO: 46) and V_{H}-CH1-hinge-CH2-CH3 heavy chain (SEQ ID NO: 45) of mAb192 (a rat-derived MG-inducing antibody), the V_{L} and V_{H} were exchanged. In the heavy chain where V_{H} was substituted with V_{L}, the junction between V_{L} and CH1-hinge-CH2-CH3 was connected via an -S-S- linker, and the CH1, CH2, and CH3 regions were converted from G1 to IgG2/4. The CL is a kappa chain. The heavy and light chains of mAb192 C IgG2/4 have the amino acid sequences of SEQ ID NO: 3 and SEQ ID NO: 4, respectively.

mAb192 B: This antibody has a V_{L}-kappa-hinge-CH2+CH3(IgG2/4) + V_{H}-CH1(IgG2/4) structure.

Specifically, mAB192 B was prepared as follows: First, in the V_{L}-CL light chain (SEQ ID NO: 46) and V_{H}-CH1-hinge-CH2-CH3 heavy chain (SEQ ID NO: 45) of mAb192, the V_{L}-CL and V_{H}-CH1 were exchanged. In the resulting V_{L}-CL-hinge-CH2-CH3 heavy chain and V_{H}-CH1 light chain, the CH1 and CH2-CH3 regions were modified to the IgG2/4 type, respectively. The heavy and light chains of mAb192 B have the amino acid sequences of SEQ ID NO: 5 and SEQ ID NO: 6, respectively.

mAb192 IgG2/4: This antibody has a human IgG2/4 structure. The CH1, CH2, and CH3 regions of the rat-derived MG-inducing antibody mAb192 were converted from IgG1 to IgG2/4. The heavy and light chains of mAb192 IgG2/4 have the amino acid sequences of SEQ ID NO: 65 and SEQ ID NO: 66, respectively.

mAb192 C IgG1: This antibody has a V_{L}-SS-IgG1 + V_{H}-kappa structure. In the light chain (SEQ ID NO: 46) and heavy chain (SEQ ID NO: 45) of mAb192, the V_{L} and V_{H} were exchanged, and in the heavy chain where V_{H} was substituted with V_{L}, the junction between V_{L} and CH1-hinge-CH2-CH3 was connected via an -S-S- linker. The CL is a kappa chain. The heavy and light chains of mAb192 C IgG1 have the amino acid sequences of SEQ ID NO: 69 and SEQ ID NO: 70, respectively.

mAb192 C IgG4: This antibody has a V_{L}-SS-IgG4 + V_{H}-kappa structure. In the light chain (SEQ ID NO: 46) and heavy chain (SEQ ID NO: 45) of mAb192, the V_{L} and V_{H} were exchanged. In the heavy chain where V_{H} was replaced with V_{L}, the junction between V_{L} and CH1-hinge-CH2-CH3 was connected via an -S-S- linker, and the CH1, CH2, and CH3 regions were converted from IgG1 to IgG4, into which mutations were introduced to inhibit Fab arm exchange. The CL is a kappa chain. The heavy and light chains of mAb192 C IgG4 have the amino acid sequences of SEQ ID NO: 71 and SEQ ID NO: 72, respectively.

Humanized mAb35 Antibody (Human IgG1): The mAb35 antibody is an autoantibody that binds to the alpha subunit of rat AChR, comprising a heavy chain of SEQ ID NO: 11 and a light chain of SEQ ID NO: 12. Since mAb35 also binds to human AChR, the CH1, CH2, and CH3 regions were converted from rat IgG to human IgG1 to create a humanized variant. The heavy and light chains of the humanized mAb35 antibody have the amino acid sequences of SEQ ID NO: 73 and SEQ ID NO: 74, respectively.

### in vitro Neuromyelitis Optica (NMO) Pathogenic Autoantibodies

rAb58: This antibody has a V_{H}-IgG1 + V_{L}-kappa structure. The rAb53 and rAb58 antibodies are autoantibodies against Aquaporin-4 (AQP4) obtained from plasmablast clones isolated from the cerebrospinal fluid of AQP4 antibody-positive patients. They bind to the AQP4 M23 isoform, causing antibody-dependent cellular cytotoxicity (ADCC) or complement-dependent cytotoxicity (CDC). rAb58 is a patient-derived antibody against AQP4, comprising a heavy chain of SEQ ID NO: 7 and a light chain of SEQ ID NO: 8. The V_{H} and V_{L} of rAb58 correspond to amino acids 1-115 of SEQ ID NO: 7 and 1-110 of SEQ ID NO: 8, respectively.

### 1-3: Recombinant Antibody According to an Embodiment for Competitively Inhibiting NMO Autoantibody rAb58 for in vitro ADCC Analysis

The rAb53 antibody has a heavy chain of SEQ ID NO: 47 and a light chain of SEQ ID NO: 48. rAb53 C: This antibody has a V_{L}-SS-IgG2/4 + V_{H}-kappa structure. rAb53 C was generated by exchanging the V_{H} and V_{L} of the rAb53 antibody, connecting the V_{L} and the CH1-hinge-CH2-CH3 junction in the resulting heavy chain via an -S-S-linker, and modifying the CH1, CH2, and CH3 regions from IgG1 to IgG2/4. rAb53 C is a recombinant antibody that competitively binds to AQP4 against the autoantibodies rAb58 or rAb53. The rAb53 C antibody comprises a heavy chain of SEQ ID NO: 9 and a light chain of SEQ ID NO: 10. The V_{H} and V_{L} of rAb53 C correspond to amino acids 1-124 of SEQ ID NO: 9 and 1-108 of SEQ ID NO: 10, respectively.

### 1-4: in vivo Myasthenia Gravis Pathogenic Autoantibody

### mAb35 Antibody; Rat IgG

The mAb35 antibody is an autoantibody that binds to the alpha subunit of rat AChR. It comprises a heavy chain of SEQ ID NO: 11 and a light chain of SEQ ID NO: 12. The V_{H} and V_{L} of mAb35 correspond to amino acids 1-121 of SEQ ID NO: 11 and 1-106 of SEQ ID NO: 12, respectively.

### 1-5: Recombinant Antibodies for in vivo Competitive Binding to Autoantigens with mAb35

mAb35 B (Competitive Recombinant Antibody of mAb35): V_{L}-kappa-CH2-CH3 + V_{H}-CH1.

mAb35 B is an IgG1 antibody in which the heavy chain V_{H}-CH1 and light chain V_{L}-CL of the mAb35 antibody were exchanged. mAb35 B comprises heavy and light chains of SEQ ID NOs: 13 and 14, respectively. The V_{H} and V_{L} of mAb35 B correspond to amino acids 1-122 of SEQ ID NO: 13 and 1-105 of SEQ ID NO: 14, respectively.

mAb35 C (Competitive Recombinant Antibody of mAb35): V_{L}-SS-CH1-CH2-CH3(IgG2/4) + V_{H}-kappa.

mAb35 C was generated by exchanging the V_{H}-CH1 and V_{L}-CL of the mAb35 antibody, connecting the V_{L} and the -CH1-hinge-CH2-CH3 junction via an -S-S- linker, and modifying the CH1, CH2, and CH3 regions from IgG1 to IgG2/4. mAb35 C comprises heavy and light chains of SEQ ID NOs: 15 and 16, respectively. The V_{H} and V_{L} of mAb35 C correspond to amino acids 1-122 of SEQ ID NO: 15 and 1-105 of SEQ ID NO: 16, respectively.

### Example 2: Competition Assay

For the competition assay, TE671 cells (human medulloblastoma-derived cells) expressing fetal acetylcholine receptor (AChR) on the cell membrane-used to validate MG-inducing antibodies-were prepared by detachment from culture dishes using 10 mM EDTA. DMEM containing FBS was used as the culture medium, and the isolated cells were prepared in PBS. Subsequently, the prepared cells were seeded into a 96-well plate at a density of 1x10⁵ cells/well. Therapeutic antibodies (competitive autoantibody inhibitors) were serially diluted to prepare various concentrations, added to the wells, and incubated at 4°C for 20 minutes to allow antigen binding. Next, without a washing step, biotinylated-mAb637 (BioLegend) was added to a concentration of 8 µg/ml and incubated at 4°C for 20 minutes to allow the therapeutic antibody and biotinylated-mAb637 to compete for the autoantigen.

Streptavidin-Allophycocyanin (SA-APC) was diluted 1:1000 in FACS buffer, added to the wells, and incubated at 4°C for 20 minutes to react with the biotinylated-mAb637. After washing the wells, fluorescence-activated cell sorting (FACS) analysis was performed on the cells to analyze the degree of competition of the therapeutic antibody with mAb637.

### Example 3: Complement-Dependent Cytotoxicity (CDC) Analysis

A recombinant 293T cell line was used for CDC analysis. This cell line was generated by introducing genes into the genome of the 293T cell line using a lentiviral system to express the extracellular domain of AChR. Specifically, four types of DNA were introduced into the 293T cells to co-express four types of AChR (A, B, D, and E).

Additionally, the CD45, CD55, and CD59 genes were knocked out in the resulting 293T cells using the CRISPR/Cas9 system. The CRISPR/Cas9 system specifically targets genomic DNA using gRNA to create nicks, leading to double-strand breakage; the cells then undergo repair via the DNA repair system with the loss of some nucleotides, causing a frame shift that knocks out normal gene expression.

These three proteins are representative complement inhibitors. Thus, recombinant cells were obtained that express the AChR-ECM gene on the cell surface and have three complement inhibitor genes knocked out. The AChR is a nicotinic-type receptor that recognizes acetylcholine (a neurotransmitter secreted from neurons) at the neuromuscular junction, opening calcium channels to signal muscle contraction. Next, the recombinant cells were detached from culture dishes using Trypsin-EDTA in DMEM (Welgene, cat. no. LB001-02) and added to 48-well plates at 1x10⁵ cells per well. Cell culture was performed in DMEM containing AOPI, glutamine, and FBS. AOPI was used to measure cell viability during culture. Normal human serum (10% v/v, Innovative Research, CISER10ML-31004) and antibodies at specified concentrations were added to the wells. The serum was intended to provide complement. The plates were incubated at 37°C for 6 hours to induce CDC. The antibodies used were disease-inducing autoantibodies (e.g., mAb35) and/or their competitive recombinant antibodies.

Subsequently, anti-C5b-9 antibody (Abcam, ab66768) was diluted 1:200 in FACS buffer (2% FBS in PBS), added to each well, and incubated at 4°C for 15 minutes. After washing each well, a secondary anti-mouse IgG2A Alexa647 antibody (R&D SYSTEMS, IC003R) and eBioscience^{™} Fixable Viability Dye eFluor^{™} 450 (FVD450, Invitrogen, 65-0863-14) for dead cell staining were diluted 1:100 in PBS buffer, added to the wells, and incubated at 4°C for 15 minutes. Following incubation, flow cytometry was performed to measure the percentage of C5b-9 positive cells and the rate of apoptosis.

### Example 4: Antibody Internalization Analysis

A recombinant 293T cell line co-expressing four types of AChR (A, B, D, and E) via a lentiviral system was used for internalization analysis. The recombinant cells were detached from dishes using Trypsin-EDTA and seeded into 96-well plates at 1x10⁴ cells per well in DMEM containing AOPI, glutamine, and FBS. To visualize antibody internalization via fluorescence, the antibodies were labeled with Incucyte Human Fabfluor-pH Red (STORIUS, Cat.no. 4722)-which fluoresces red when internalized into the lower pH environment of the cell-by mixing the antibody and reagent at a 1:2 molar ratio and incubating at 37°C for 15 minutes.

Subsequently, 4 µg/ml of Fabfluor-labeled mAb192 C IgG2/4 (and its variants) or 4 µg/ml of Fabfluor-labeled humanized mAb35 antibody, along with various concentrations of serially diluted mAb192 C IgG2/4 and its variants, were added to the wells. The cells were incubated at 37°C for 24 hours, and fluorescence was measured at 30-minute intervals using an Incucyte instrument to monitor antibody internalization.

### Example 5: in vivo Efficacy Test

To induce myasthenia gravis (MG), 1.5 mg/kg of mAb35 antibody mixed in PBS was administered intraperitoneally to 4-week-old Lewis rats, which are used as an autoimmune disease-inducing model. The Lewis rat is a strain derived from the Wistar rat, characterized by white fur, and is susceptible to obesity induction by high-fat diets and autoimmune myocarditis. Lewis rats are widely used as models for multiple sclerosis and autoimmune diseases and are the most commonly used strain for inducing myasthenia gravis. The degree of MG induction was measured by monitoring the body weight and clinical scores of the subjects. The mAb35 antibody is a rat-derived autoantibody against rat AChR and is a pathogenic antibody that induces MG when passively transferred into rats. Symptoms were observed in subjects administered with the mAb35 antibody, and the test was conducted for up to 48 hours to obtain experimental rats in which MG was induced.

The clinical scores were assigned as follows: after performing exercise several times by grasping the wire mesh of the cage, the post-exercise state was checked and scored on a 5-step scale. Grade 0: Normal muscle activity after exercise. Grade 1: Unable to lift the head with the chin on the floor after exercise, hunched back, and reduced mobility. Grade 2: Resting without movement after exercise. Grade 3: Symptoms of severe muscle weakness (paralysis, loss of gripping ability) and dyspnea during exercise. Grade 4: Death.

To confirm the therapeutic and preventive effects of the antibody according to the present invention against autoimmune diseases, each therapeutic antibody was administered intravenously before the administration of the mAb35 antibody, i.e., prior to the induction of MG. Generally, clinical symptoms of MG appear 24 hours after the administration of an autoantibody such as mAb35. The therapeutic antibody was administered ahead of mAb35 to confirm not only therapeutic but also preventive utility. Depending on the experimental design, the therapeutic antibody was administered as a single dose or repeated four times. Blood was collected via jugular vein sampling at regular intervals before and after the administration of the therapeutic antibody or the disease-inducing antibody mAb35.

After the collected blood was coagulated, serum was separated, and the concentration of the therapeutic or pathogenic antibody in the serum was measured via ELISA. In addition, the tibialis anterior (TA) muscle of the rat was isolated and imaged through ELISA and immunostaining. This was to confirm whether the antibody binds to the rat muscle AChR and to verify the antibody concentration in the muscle.

### Example 6: ELISA for AChR

The AChR used in the present invention was derived from the Pacific electric ray (Torpedo californica). Specifically, an AChR-enriched membrane fraction obtained from the electric organ of Torpedo californica was added to each well of an ELISA plate at 5 to 10 µg/ml in PBS and incubated overnight at 4°C to coat the wells with AChR. After washing the wells three times with washing buffer, a buffer containing 2% BSA was added and incubated at room temperature for 1 hour to block the surface. Subsequently, samples were diluted to appropriate concentrations, added to the wells, and incubated at 37°C for 2 hours. Human serum or rat tibialis anterior lysate (TA lysate) was used as the sample. After washing the wells five times with washing buffer, anti-rat HRP or antihuman HRP antibodies diluted 1:1000 to 1:5000 were added and incubated at 37°C for 1 hour. After washing five times with washing buffer, TMB substrate was added and incubated for 15 minutes. The reaction was stopped by adding 2N $H_2SO_4$, and the absorbance was measured at 450 nm. HRP stands for horseradish peroxidase, and TMB stands for 3,3',5,5'-tetramethylbenzidine.

### Example 7: in vitro Efficacy for Myasthenia Gravis and Neuromyelitis Optica

### 7-1: Competitive Analysis

The present inventors analyzed the binding competition between autoantibodies and recombinant antibodies for autoantigens. Specifically, the competitive binding of the autoantibody mAb637 against the autoantigen AChR and its competitive recombinant antibodies mAb637 B and mAb637 C was analyzed. The autoantibody is mAb637, which induces MG in humans, and the recombinant antibodies are mAb637 B, mAb637 C, and mAb192 C IgG2/4, while the autoantigen is AChR. As shown in FIG. 3, mAb637 B has a structure where the V_{H}-CH1 of the heavy chain and the V_{L}-kappa of the light chain of mAb637 are exchanged (i.e., it has a heavy chain structure of V_{L}-kappa-CH2-CH3 and a light chain structure of V_{H}-CH1), and mAb637 C has a structure where the V_{H} of the heavy chain and the VL of the light chain of mAb637 are exchanged (i.e., it has a heavy chain structure of V_{L}-CH1-CH2-CH3 and a light chain structure of V_{H}-kappa). Specifically, a biotinylated pathogenic antibody and native recombinant antibodies were used, and the pathogenic antibody was detected with Alexa647-conjugated streptavidin. After analyzing the signal intensity through a fluorescence-activated cell sorter (FACS), the signal intensity when the pathogenic antibody was 100% bound was set to 100, and the intensity when the candidate antibody was 100% bound was substituted with 0. The degree of signal decrease was observed when the binding affinity of the pathogenic antibody was inhibited by the candidate antibody. Specifically, the candidate antibody was diluted by concentration to observe changes in the signal. FIG. 3 shows the results of confirming the competitive binding ability to the antigen between the biotinylated pathogenic antibody and the native candidate antibody. The pathogenic antibody was detected with Alexa647-conjugated streptavidin, and the signal intensity was analyzed via FACS; the signal intensity when the pathogenic antibody was 100% bound was set to 100, while the intensity when the candidate antibody was 100% bound was set to 0 to observe the degree of signal reduction when the binding affinity of the pathogenic antibody was inhibited.

As a result, as shown in FIG. 3, both mAb637 B and mAb637 C antibodies competitively bound to the autoantigen against mAb637, interfering with the binding of mAb637 to the autoantigen. These results suggest that the antibody of the present invention can prevent or treat autoimmune diseases by competitively inhibiting antibodies that bind to autoantigens and cause a decrease in autoantigen function.

### 7-2: Complement-Dependent Cytotoxicity (CDC) Assay

The present inventors contacted 293T cell lines expressing the AChR gene and having three complement inhibitor genes knocked out with the autoantibody humanized mAb35, or humanized mAb35 combined with the competitive recombinant antibody mAb192 C IgG2/4, respectively, to confirm the degree of CDC induction *in vitro.*

Specifically, the cell lines were contacted with the pathogenic humanized mAb35 antibody and the competitive recombinant antibody mAb192 C IgG2/4, followed by the addition of normal human serum and complement, and incubation at 37°C for 6 hours to induce CDC. After washing the cells, anti-C5b-9 antibody was added and incubated, and the ratio of C5b-9 positive cells and the cell death rate (after adding FVD450) were measured using a FACS. Then, when 293T cells expressing the AChR gene and lacking three complement inhibitor genes were contacted with the pathogenic antibody humanized mAb35, or humanized mAb35 and the competitive recombinant antibody mAbl92 C IgG2/4, the ratio of induced C5b-9 positive cells and dead cells was measured.

The concentration of the pathogenic antibody was 0 µg/ml on the left and 10 µg/ml for all subsequent samples; the concentration of mAb192 C IgG2/4 was 0.00 µg/ml, 0.00 µg/ml, 0.04 µg/ml, 0.15 µg/ml, 0.62 µg/ml, 2.5 µg/ml, and 10 ug/ml from left to right; and complement was added as 10% human serum. Pathogenic antibody refers to serum containing humanized mAb35. mAb192 C IgG2/4 refers to a recombinant antibody containing the light chain of SEQ ID NO: 3 and the heavy chain of SEQ ID NO: 4.

As a result, treatment with humanized mAb35 increased the ratio of C5b-9 positive cells (FIG. 4a) and the ratio of cells killed by CDC (FIG. 4b) in a concentration-dependent manner, whereas co-treatment with the competitive recombinant antibody mAb192 C IgG2/4 suppressed the increase in C5b-9 positive cells and inhibited the cell death rate. Additionally, when 293T cells expressing the AChR gene and lacking three complement inhibitor genes were contacted with the autoantibody humanized mAb35 and either the competitive recombinant antibody mAb192 C IgG2/4 or its variants (mAb192 C IgG1 and mAb192 C IgG4), the inhibition rate of induced C5b-9 positive cells was analyzed. The CDC inhibition capacity was calculated by setting the case where only the pathogenic antibody and complement were added (without mAb192 C IgG2/4 or variants) as 100% induction and the case with only complement as 0%; the relative signal by mAb192 C IgG2/4 and the variants was then calculated to represent the degree of CDC inhibition.

As a result, as shown in FIGs. 4c and 4d, when co-treated with 0.15 µg/ml of mAb192 C IgG2/4 (where V_{L} and V_{H} were exchanged and CH1, CH2, and CH3 regions were changed from IgG1 to IgG2/4) or its variants, the increased ratio of C5b-9 positive cells and apoptosis caused by humanized mAb35 treatment decreased by more than about 60% and 37%, respectively. These results demonstrate the excellent CDC inhibitory capacity of the candidate antibody with exchanged V_{L} and V_{H}.

### 7-3: Analysis of ADCC Inhibitory Capacity of Recombinant Antibodies Using a Neuromyelitis Optica Cell Model

Neuromyelitis optica (NMO) spectrum disorder, also known as Devic disease, is a chronic disease of the brain and spinal cord where inflammation of the optic nerve (optic neuritis) and inflammation of the spinal cord (myelitis) result in damage or destruction of the myelin and underlying nerve fibers. NMO is known to be induced by ADCC mediated by autoantibodies against Aquaporin-4. To confirm the inhibitory capacity of the therapeutic antibody against NMO, the present inventors established an *in vitro* ADCC model using an Aquaporin-4 CHOK1 (AQP4-CHOK1) cell line expressing pathogenic Aquaporin-4. First, model cells were cultured in the presence of NMO pathogenic autoantibodies and recombinant antibodies that competitively bind to Aquaporin-4, and the degree of ADCC occurrence was measured. Specifically, AQP4-CHOK1, pathogenic antibodies (alone or with the present antibody), and NK92 cells were co-cultured for 1 hour to measure the viability of AQP4-CHOK1 cells killed by NK92 cells. The NMO pathogenic autoantibody used was rAb58, an antibody against rat-derived Aquaporin-4. The recombinant antibody that competitively binds to Aquaporin-4 is rAb53 C. The rAb53 C antibody has a structure in which the V_{H} domain of the heavy chain and the V_{L} domain of the light chain of the rAb53 antibody are exchanged, and the heavy chain constant region is substituted from IgG1 to IgG2/4.

FIG. 5 of the present invention shows the results of analyzing cell viability when Aquaporin-4-expressing U87MG cells were contacted with the NMO pathogenic autoantibody rAb58 or the recombinant antibody rAb53 C, which competitively binds to the Aquaporin-4 antigen. For the cases where cells were contacted with rAb58, rAb53 C, or rAb58 after rAb53 C, the relative cell viability was calculated by setting the viability of AQP4-CHOK1 cells co-incubated only with NK92 cells (without antibodies) as 100% and the case of complete death as 0%. The cell viability ratio upon adding each antibody was then calculated and plotted.

As a result, as shown in FIG. 5, when cells were contacted with rAb58, the relative viability decreased according to concentration, but when contacted with rAb53 C, the relative viability tended to be maintained. Furthermore, when cells were contacted with rAb58 after rAb53 C, the decrease in relative cell viability tended to be suppressed. These results appear to be because rAb53 C binds to the autoantigen and prevents the autoantibody from binding, thereby reducing cell internalization, and because ADCC does not occur due to rAb53 C having its effector functions removed.

### 7-4: Internalization Assay

The present inventors contacted 293T cell lines expressing the AChR gene with Fabfluor-labeled pathogenic substances or candidate antibody variants (mAb192 C IgG1, mAb192 C IgG4, and mAb192 C IgG2/G4) to confirm the degree of internalization induction *in vitro.* Specifically, 4 µg/ml of Fabfluor-labeled pathogenic antibody mAb192 IgG1 and competitive recombinant antibody mAb192 C IgG2/4 and its variants (mAb192 C IgG1 and mAb192 C IgG4) were respectively contacted with the cell lines and incubated at 37°C for 24 hours, measuring the degree of antibody internalization via fluorescence at 30-minute intervals. Additionally, Fabfluor-labeled humanized mAb35 antibody and various concentrations of serially diluted mAb192 C IgG2/4 and its variants were respectively contacted and incubated at 37°C for 24 hours, measuring the internalization degree via fluorescence at 30-minute intervals.

As a result, as shown in FIG. 6a, the highest level of internalization occurred when the cells were contacted with mAb192; whereas, for the substances in which the V_{L} and V_{H} were exchanged (mAb192 C IgG1 and mAb192 C IgG4) or in which the V_{L} and V_{H} were exchanged along with the replacement of the CH1, CH2, and CH3 regions with IgG2/4 (mAb192 C IgG2/4), internalization was found to decrease compared to the pathogenic substance. In particular, among the variants with exchanged V_{L} and V_{H}, the competitive recombinant antibody mAb192 C IgG2/4-which also featured the CH1, CH2, and CH3 regions exchanged for IgG2/4-exhibited the lowest degree of internalization, suggesting that the possibility of AChR internalization induced by antibody binding is also extremely low.

FIGs. 6b to 6d show the levels to which the internalization of the pathogenic humanized mAb35 antibody is inhibited by the competitive recombinant antibody mAb192 C IgG2/4 and its variants, mAb192 C IgG1 and mAb192 C IgG4.

FIG. 6b demonstrates that the inhibition of humanized mAb35 internalization increases as the concentration of mAb192 C IgG2/4 or its variants increases; at a concentration of 40 µg/ml, mAb192 C IgG2/4 or its variants inhibited the internalization of most of the pathogenic substance. In particular, the recombinants with exchanged V_{L} and V_{H} exhibited an excellent inhibitory effect on the internalization of the pathogenic substance, indicating that the form in which V_{L} and V_{H} are exchanged is effective in suppressing the internalization of pathogenic materials. Taken together, FIGs. 6a to 6d show that mAb192 C IgG2/4-in which V_{L} and V_{H} are exchanged and the CH1, CH2, and CH3 regions are replaced with IgG2/4-does not undergo significant internalization itself and effectively inhibits the internalization of pathogenic substances. This demonstrates that it is the most optimal form for suppressing the loss of AChR, which is otherwise caused by the pathogenic substance binding to and co-internalizing with the receptor.

### Example 8: Myasthenia Gravis in vivo Efficacy Test

### 8-1: in vivo Efficacy of Competitive Recombinant Antibodies

Lewis rats were administered 5 mg/kg of the therapeutic recombinant antibody four times at 3.5-day intervals, followed by 1.5 mg/kg of the MG pathogenic autoantibody, and changes in body weight and MG clinical scores over time were monitored. The pathogenic autoantibody was mAb35, and the therapeutic recombinant antibody was one in which the V_{H} domain of the heavy chain and the V_{L} domain of the light chain of mAb35 were exchanged, and the CH2-CH3 domains of the heavy chain constant region were substituted with IgG2/4.

FIG. 7a shows the body weight change over time after administering 5 mg/kg of the therapeutic antibody four times at 3.5-day intervals followed by 1.5 mg/kg of MG pathogenic autoantibody mAb35. FIG. 7b shows the clinical score change over time for the same conditions. FIG. 7c shows the body weight and clinical score changes by dosage of the therapeutic antibody. In FIGs. 7a and 7b, G1 received only the vehicle PBS, G2 received mAb35 after PBS, G3 received the therapeutic antibody, and G4 received both mAb35 and the therapeutic antibody. As shown in FIGs. 7a and 7b, in the group administered with only PBS (G1), weight loss due to administration stress was observed, but overall weight tended to increase during the period. In the group administered with mAb35 after PBS (G2), body weight decreased and MG pathology was confirmed to be induced. In the group repeatedly administered with the therapeutic antibody (G3), weight gain similar to the PBS group was observed, and no MG pathology induction was found. In the group administered with both (G4), no body weight loss or MG pathology by mAb35 appeared at all. This indicates that the therapeutic antibody not only fails to induce pathology but also has a preventive effect against pathology induced by autoantibodies.

When mAb35 was administered after repeated therapeutic antibody doses, body weight decreased sharply only in the group that did not receive the therapeutic antibody, while there was no significant change in the group that did. Similarly, clinical scores appeared only in the group not receiving the therapeutic antibody, and some subjects eventually died (clinical score=4). Clinical scores were not measured in any group except the mAb35-administered group (G2), thus they are not displayed in FIG. 7b.

Furthermore, as shown in FIG. 7c, to confirm the dose-dependent effect, therapeutic antibodies at 0 mg/kg, 0.125 mg/kg, 0.25 mg/kg, 0.5 mg/kg, 1 mg/kg, and 2 mg/kg were administered four times over two weeks; inhibition of symptoms and weight loss by mAb35 was observed starting from the 0.25 mg/kg dose, and no clinical symptoms were observed at concentrations of 0.5 mg/kg or higher.

According to the above results, it is confirmed from the aforementioned examples that the antibody of the present invention is effective in treating autoimmune diseases associated with a decrease in autoantigen function. In particular, the antibody platform of the present invention exhibits excellent effects in preventing or treating autoimmune diseases induced by autoantibodies such as mAb35, mAb637, mAb192, or rAb53, confirming its utility for various autoimmune diseases. Furthermore, the platform has been confirmed to have preventive or therapeutic effects on various diseases such as myasthenia gravis or neuromyelitis optica, and thus can be utilized for the prevention or treatment of diverse types of autoimmune diseases.

The present invention has been described with reference to the above-described embodiments, but these are merely exemplary, and those of ordinary skill in the art will understand that various modifications and equivalent other embodiments are possible. Therefore, the true technical protection scope of the present invention should be determined by the technical spirit of the appended claims.

## Claims

1. A recombinant homodimeric antibody derived from an autoantibody having specificity for an autoantigen and comprising a light chain and a heavy chain, wherein the recombinant homodimeric antibody possesses specificity for said autoantigen, wherein the heavy chain comprises at least a partial region of a light chain variable region (V_{L}) of the autoantibody, and the light chain comprises at least a partial region of a heavy chain variable region (V_{H}) of the autoantibody.

2. The homodimeric antibody according to claim 1, wherein the autoantigen is a membrane protein.

3. The homodimeric antibody according to claim 1, which reduces or prevents the formation of a cross-linking structure through the binding of the autoantibody with a plurality of the autoantigens.

4. The homodimeric antibody according to claim 3, which reduces or prevents internalization of an antigen-antibody complex caused by the cross-linking.

5. The homodimeric antibody according to claim 3, wherein the autoantibody induces complement binding through the cross-linking.

6. The homodimeric antibody according to claim 1, which inhibits binding of the autoantibody to the autoantigen by binding to the same site as an epitope of the autoantigen to which the autoantibody binds or to a site proximate to said epitope.

7. The homodimeric antibody according to claim 1, wherein a binding orientation of at least one monomer of the homodimeric antibody relative to the autoantigen is different from a binding orientation of the autoantibody relative to the autoantigen.

8. The homodimeric antibody according to claim 7, wherein the binding of one monomer of the homodimeric antibody to a first autoantigen results in a binding orientation that prevents the remaining monomer of the homodimeric antibody from binding to a second autoantigen present on a cell surface.

9. The homodimeric antibody according to claim 7, having a conformation that does not bind to a plurality of complex autoantigens among complex autoantigens present on a cell surface.

10. The homodimeric antibody according to claim 9, which does not cross-link two or more complex autoantigens present on the cell surface.

11. The homodimeric antibody according to claim 1, wherein an Fc region of the heavy chain is an IgG1 Fc, an IgG4 Fc, or an IgG2/4 hybrid Fc.

12. The homodimeric antibody according to claim 1, wherein the autoantibody comprises a first half-antibody fragment comprising a first light chain variable region (V_{L}) and a first heavy chain variable region (V_{H}) and having specificity for an autoantigen, and a second half-antibody fragment comprising a second light chain variable region (V_{L}) and a second heavy chain variable region (V_{H}) and having specificity for the autoantigen;
wheirein the homodimeric antibody comprises at least one of the following half-antibody fragments:
i) a heavy chain comprising the first light chain variable region (V_{L}), and a light chain comprising the first heavy chain variable region (V_{H});
ii) a heavy chain comprising the second light chain variable region (V_{L}), and a light chain comprising the second heavy chain variable region (V_{H});
iii) a heavy chain comprising at least one of CDRL1, CDRL2, and CDRL3 of the first light chain variable region (V_{L}), and a light chain comprising at least one of CDRH1, CDRH2, and CDRH3 of the first heavy chain variable region (V_{H}) and the remaining CDRL portions not included in the heavy chain; and
iv) a heavy chain comprising at least one of CDRL1, CDRL2, and CDRL3 of the second light chain variable region (V_{L}), and a light chain comprising at least one of CDRH1, CDRH2, and CDRH3 of the second heavy chain variable region (V_{H}) and the remaining CDRL portions not included in the heavy chain.

13. The homodimeric antibody according to claim 1, wherein partial regions of the variable regions of the heavy chain and the light chain of the autoantibody are exchanged with each other.

14. The homodimeric antibody according to claim 1, wherein the autoantibody comprises a first half-antibody fragment comprising a first light chain variable region and a first heavy chain variable region and having specificity for the autoantigen; and a second half-antibody fragment comprising a second light chain variable region and a second heavy chain variable region and having specificity for the autoantigen, wherein in the homodimeric antibody, a V_{L}-CL and a V_{H}-CH1 of at least one half-antibody fragment among the half-antibody fragments of the autoantibody are exchanged with each other.

15. A pharmaceutical composition for preventing or treating an autoimmune disease, comprising the homodimeric antibody according to any one of claims 1 to 13 and a pharmaceutically acceptable carrier.

16. A recombinant dimeric antibody comprising a light chain variable region and a heavy chain variable region and having specificity for an autoantigen,
wherein in at least one monomer of the recombinant dimeric antibody, at least a partial region of a light chain variable region (V_{L}) of an autoantibody, which has specificity for the autoantigen and pathogenicity for an autoimmune disease, and at least a partial region of a heavy chain variable region (V_{H}) of said autoantibody are exchanged with each other.
